(19) Europäisches Patentamt
European Patent Office
Office européen des brevets

(11) EP 3 044 818 B1

(12) EUROPÄISCHE PATENTSCHRIFT

(45) Veröffentlichungstag und Bekanntmachung des Hinweises auf die Patenterteilung:
**13.11.2019 Patentblatt 2019/46**

(21) Anmeldenummer: **14781089.9**

(22) Anmeldetag: **12.09.2014**

(51) Int Cl.:
***H01L 51/46*** *(2006.01)* *C07D 471/06* *(2006.01)*

(86) Internationale Anmeldenummer:
**PCT/EP2014/069478**

(87) Internationale Veröffentlichungsnummer:
**WO 2015/036529 (19.03.2015 Gazette 2015/11)**

# (54) VORRICHTUNG DER ORGANISCHEN ELEKTRONIK MIT AKTIVER SCHICHT

ORGANIC ELECTRONIC DEVICE WITH ACTIVE LAYER

DISPOSITIF DE L'ÉLECTRONIQUE ORGANIQUE À COUCHE ACTIVE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **13.09.2013 EP 13184372**

(43) Veröffentlichungstag der Anmeldung:
**20.07.2016 Patentblatt 2016/29**

(73) Patentinhaber: **Heliatek GmbH**
**01139 Dresden (DE)**

(72) Erfinder:
- **WEISS, Andre**
**01139 Dresden (DE)**
- **UHRICH, Christian**
**01139 Dresden (DE)**
- **FISCHER, Georg**
**78457 Konstanz (DE)**
- **DALTROZZO, Ewald**
**78467 Konstanz (DE)**
- **ZUMBUSCH, Andreas**
**78457 Konstanz (DE)**
- **TANABE, Junichi**
**Takarazuka**
**Hyogo 665-0847 (JP)**
- **HWANG, Jae Hyung**
**68519 Viernheim (DE)**
- **BRUDER, Ingmar**
**67271 Neuleiningen (DE)**
- **ERK, Peter**
**67227 Frankenthal (DE)**
- **SENS, Rüdiger**
**67069 Ludwigshafen (DE)**

(56) Entgegenhaltungen:
**EP-A1- 2 272 849**

- **SIMON WIKTOROWSKI ET AL: "Photophysics of aminophenyl substituted pyrrolopyrrole cyanines", PHYSICAL CHEMISTRY CHEMICAL PHYSICS, Bd. 14, Nr. 8, 1. Januar 2012 (2012-01-01) , Seite 2921, XP55151839, ISSN: 1463-9076, DOI: 10.1039/c2cp23330d**
- **G. M. FISCHER, E. DALTROZZO, A. ZUMBUSCH: ANGEW. CHEM. INT. ED., Bd. 50, 5. Januar 2011 (2011-01-05), Seiten 1406-1409, XP55057949, in der Anmeldung erwähnt**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft eine Vorrichtung der organischen Elektronik, die mindestens zwei Elektroden und mindestens eine zwischen den Elektroden liegende organische Schicht umfasst, wobei die organische Schicht wenigstens eine Pyrrolopyrrol-Cyanin-Verbindung enthält.

Es wird erwartet, dass zukünftig in vielen Bereichen der Elektronikindustrie neben den klassischen anorganischen Halbleitern zunehmend auch organische Halbleiter auf Basis von niedermolekularen oder polymeren Materialien eingesetzt werden. Diese weisen vielfach Vorteile gegenüber den klassischen anorganischen Halbleitern auf, beispielsweise eine bessere Substratkompatibilität und eine bessere Verarbeitbarkeit der auf ihnen basierenden Halbleiterbauteile. Sie erlauben die Verarbeitung auf flexiblen Substraten. Die organische Elektronik beschäftigt sich schwerpunktmäßig mit der Entwicklung neuer Materialien und Fertigungsprozesse für die Herstellung elektronischer Bauelemente auf der Basis organischer Halbleiterschichten. Dazu zählen vor allem organische Leuchtdioden (organische lichtemittierende Dioden, organic light emitting diods, OLED), schaltende Elemente wie organische Transistoren, z. B. organische Feldeffekttransistoren (Organic Field-Effect Transistors, OFET) und organische TFT (Thin Film Transistor) sowie die organische Photovoltaik.

Durch den Einsatz geeigneter neuartiger organischer Materialien können verschiedene neuartige Bauelemente, die auf organischer Elektronik beruhen, wie Displays. Sensoren, Transistoren, Datenspeicher oder Photovoltaik-Zellen bereitgestellt werden. Dadurch ist die Entwicklung neuer Anwendungen, die dünn, flexibel, leicht und zu niedrigen Kosten herstellbar sind, möglich.

Ein bevorzugter Anwendungsbereich der organischen Elektronik ist der Einsatz in Vorrichtungen wie schaltende Elemente, organische Solarzellen und Photodetektoren sowie Elektrolumineszenz-Bauelemente.

Organischen Feldeffekttransistoren wird ein großes Entwicklungspotential, beispielsweise in Speicherelementen und integrierten optoelektronischen Vorrichtungen, zugeschrieben. In organischen lichtemittierenden Dioden (OLED) wird die Eigenschaft von Materialien ausgenutzt, Licht zu emittieren, wenn sie durch elektrischen Strom angeregt werden. OLEDs sind insbesondere interessant als Alternative zu Kathodenstrahlröhren und Flüssigkristalldisplays zur Herstellung von Flachbildschirmen. Aufgrund der sehr kompakten Bauweise und des intrinsisch niedrigeren Stromverbrauchs eignen sich Vorrichtungen, die OLEDs enthalten, insbesondere für mobile Anwendungen, zum Beispiel für Anwendungen in Mobiltelefonen, Laptops, Digitalkameras, MP3-Playern usw. sowie zur Beleuchtung.

Die Direktumwandlung von Solarenergie in elektrische Energie in Solarzellen beruht auf dem inneren Photoeffekt eines Halbleitermaterials, d.h. der Erzeugung von Elektron-Loch-Paaren durch Absorption von Photonen und der Trennung der negativen und positiven Ladungsträger an einem p-n-Übergang oder einem Schottky-Kontakt. Die so erzeugte Photospannung kann in einem äußeren Stromkreis einen Photostrom bewirken, durch den die Solarzelle ihre Leistung abgibt.

Vom Halbleiter können dabei nur solche Photonen absorbiert werden, die eine Energie aufweisen, die größer als seine Bandlücke ist. Die Größe der Halbleiterbandlücke bestimmt also den Anteil des Sonnenlichts, der in elektrische Energie umgewandelt werden kann. Es wird zukünftig erwartet, dass organische Solarzellen die klassischen Solarzellen auf Siliziumbasis aufgrund geringerer Kosten, eines geringeren Gewichts, der Möglichkeit zur Herstellung flexibler und/oder farbiger Zellen, der besseren Möglichkeit zur Feinabstimmung des Bandabstands übertreffen werden. Es besteht somit ein großer Bedarf an organischen Halbleitern, die sich zur Herstellung organischer Solarzellen eignen.

Um die Sonnenenergie möglichst effektiv zu nutzen, bestehen organische Solarzellen normalerweise aus zwei absorbierenden Materialien mit unterschiedlicher Elektronenaffinität bzw. unterschiedlichem Ionisationsverhalten. Das eine Material wirkt dann als p-Leiter (Elektronendonor), das andere als n-Leiter (Elektronenakzeptor). Die ersten organischen Solarzellen bestanden aus einem zweilagigen System aus einem KupferPhthalocyanin (CuPc) als p-Leiter und Perylen-3,4:9,10-tetracarbonsäurebisimidazol (PTCBI) als n-Leiter und zeigten einen Wirkungsgrad von 1 %. Um möglichst alle auftreffenden Photonen zu nutzen, werden relativ hohe Schichtdicken eingesetzt (z. B. 100 nm). Um Strom zu erzeugen, muss der durch die absorbierten Photonen erzeugte angeregte Zustand jedoch eine p-n-Grenzschicht ("p-n-junction") erreichen, um ein Loch und ein Elektron zu erzeugen, welches dann zur Anode und Kathode fließt. Die meisten organischen Halbleiter haben jedoch nur Diffusionslängen für den angeregten Zustand von bis zu 10 nm. Selbst durch die besten bisher bekannten Herstellverfahren kann die Distanz, über die der angeregte Zustand weitergeleitet werden muss, auf minimal 10 bis 30 nm verringert werden.

Neuere Entwicklungen in der organischen Photovoltaik gehen in die Richtung der sogenannten "Bulk-Heterojunction": Die photoaktive Schicht enthält hierbei die Akzeptor- und Donorverbindung(en) als bikontinuierliche Phase. Durch photoinduzierten Ladungstransfer vom angeregten Zustand der Donorverbindung zur Akzeptorverbindung findet aufgrund der räumlichen Nähe der Verbindungen eine, verglichen mit anderen Relaxationsvorgängen, schnelle Ladungstrennung statt und die entstandenen Löcher und Elektronen werden über die entsprechende Elektroden abgeführt. Zwischen die Elektroden und die photoaktive Schicht werden oftmals weitere Schichten, wie z. B. Löcher- oder Elektronentransportschichten, aufgebracht, um die Effizienz solcher Zellen zu erhöhen.

Bislang werden als Donormaterialien in solchen Bulk-Heterojunction-Zellen meist Polymere, wie z. B. Polyvinylphenylene oder Polythiophene, oder Farbstoffe aus der Klasse der Phthalocyanine, z. B. Zn- oder Vanadylphthalocyanine, und als Akzeptormaterialien Fulleren und Fulleren-Derivate sowie verschiedene Perylene verwendet.

Die Charakterisierung einer Solarzelle erfolgt in der Regel mit Hilfe von drei Kenngrößen: der Leerlaufspannung $V_{OC}$, dem Kurzschlussstrom $I_{SC}$ und dem Füllfaktor FF (FF = ($V_{mpp}$ $I_{mpp}$ / $V_{OC}$ $I_{SC}$). Zwischen Leerlauf und Kurzschluss existiert ein Punkt auf der Kennlinie (Strom als Funktion der Spannung) der Solarzelle, in dem die abgegebene Leistung maximal ist (mpp, maximum power point). Weiterhin von Interesse sind die interne und externe Quanteneffizienz und der Wirkungsgrad (η). Die interne Quanteneffizienz ist das Verhältnis aus der Anzahl an den Kontakten extrahierter Ladungsträger zur Anzahl absorbierter Photonen. Die externe Quanteneffizienz ist das Verhältnis aus der Anzahl an den Kontakten extrahierter Ladungsträger zur Anzahl eingestrahlter Photonen. Eine gute Solarzelle sollte möglichst dort eine hohe externe Quanteneffizienz aufweisen, wo die Intensität des Sonnenlichts besonders hoch ist. Der Wirkungsgrad (η) einer Solarzelle berechnet sich aus dem Verhältnis der maximalen photovoltaisch erzeugten Leistung zu der entsprechenden eingestrahlten Lichtleistung ($P_{Licht}$):

$$\eta = (V_{mpp} \bullet\ I_{mpp} / P_{Licht}) = FF \bullet\ V_{OC} \bullet\ I_{SC} / P_{Licht}$$

Bisher wurde in Vorrichtungen der organischen Elektronik in der Regel der sichtbare Spektralbereich im Wellenlängenbereich von 400 bis 650 nm genutzt. Damit bleibt ein großer Teil des Sonnenlichtes, das im nahen Infrarot-Spektralbereich (NIR) liegt, ungenutzt. Im Rahmen dieser Anmeldung versteht man unter dem Begriff "nahes Infrarot (NIR)" den Wellenlängen, der sich an den sichtbaren Bereich anschließt und im Bereich von 650 bis 1400 nm liegt.

Im Vergleich zum ultravioletten/sichtbaren Spektralbereich sind nur wenige Farbstoffklassen bekannt, die eine Lichtabsorption im roten bis infraroten Spektralbereich aufweisen und in der organischen Elektronik eingesetzt werden. WO 2006/111511 betrifft Hexarylen- und Pentarylentetracarbonsäurediimide als Aktivkomponenten in der Photovoltaik. WO 2007/116001 betrifft flüssigkristalline Rylentetracarbonsäurederivate und deren Verwendung als organische Halbleiter vom n-Typ zur Herstellung von organischen Feldeffekttransistoren und von Solarzellen. WO 2008/145172 betrifft substituierte Carboxyphthalocyanine und deren Verwendung als Aktivkomponente in der Photovoltaik.

Eine weitere Klasse von NIR-Farbstoffen sind Pyrrolopyrrol-Cyanine. Pyrrolopyrrol-Cyanine und ihre Herstellung sind beispielsweise in Angew. Chem., 119 (2007) 3824-3827 (Angew. Chem. Int. Ed. 46 (2007) 3750-3753), Angew. Chem. 2011, 123, 1442-1445 (Angew. Chem. Int. Ed., 50 (2011) 1406-1409), Eur. J. Org. Chem. 2011, 3421-3429, Chem. Comm., 46 (2010), 5289-5291 und Chem. Eur. J., 15 (2009) 4857-4864 beschrieben. Sie sind für verschiedenste Anwendungen vorgeschlagen worden, beispielsweise als NIR-Fluorophor in der Fluoreszenzspektroskopie. EP 2 272 849 A1 beschreibt Pyrrolopyrrol-Farbstoffe, genauso wie Phys.Chem.Chem. Phys., (14) 2012, 2921-2928 die Eigenschaften von Pyrroloporrol-Cyaninen mit verschiedenen Aminophenyl-Substituenten beschrebit. Der Einsatz von Pyrrolopyrrol-Cyaninen in Vorrichtungen der organischen Elektronik ist bislang nicht beschrieben.

Die aus dem Stand der Technik bekannten NIR-Farbstoffe sind hinsichtlich ihrer Absorption, der Bandlücke zwischen HOMO- und LUMO-Energieniveaus und der Prozessierbarkeit im Vakuum sowie der Transporteigenschaften wie Lochtransport, Elektronentransport und Excitonentransport teilweise nicht zufriedenstellend oder die NIR-Farbstoffe besitzen unerwünschte Eigenschaften, wie eine zu geringe Fotostabilität.

Der vorliegenden Erfindung liegt die Aufgabe zugrunde, weitere Farbstoffe, die im NIR-Bereich absorbieren, zur Verwendung in Vorrichtungen der organischen Elektronik, insbesondere zur Verwendung in organischen Solarzellen, OLEDs, Fotodetektoren oder schaltende Elementen wie organische Transistoren, bereitzustellen. Die NIR-Absorber sollen synthetisch gut zugänglich und fotostabil sein. Des Weiteren sollen die NIR-Absorber sich zur Herstellung von vakuum-prozessierbaren Solarzellen eignen. Insbesondere ist es Aufgabe der vorliegenden Anmeldung NIR-Farbstoffe bereitzustellen, die für die Umwandlung von Lichtenergie in elektrische Energie in Vorrichtungen der organischen Elektronik einen ausreichenden Wirkungsgrad besitzen.

Diese Aufgabe wird gelöst durch die Bereitstellung einer Vorrichtung der organischen Elektronik, umfassend mindestens zwei Elektroden und mindestens eine zwischen den

Elektroden liegende organische Schicht, wobei die organische Schicht wenigstens eine Pyrrolopyrrol-Cyanin-Verbindung der Formel I enthält

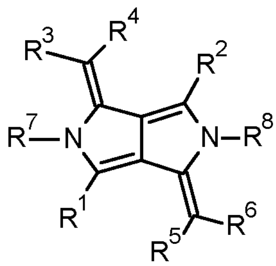

(I)

worin

$R^1$ und $R^2$ unabhängig voneinander ausgewählt sind unter Wasserstoff, $C_1$-$C_{20}$-Alkyl, $C_1$-$C_{20}$-Alkoxy, $C_3$-$C_{15}$-Cycloalkyl, $C_3$-$C_{15}$-Cycloalkenyl, $C_3$-$C_{15}$-Cycloalkyloxy, 3-bis 15-gliedrigem gesättigten oder partiell ungesättigten Heterocyclus, 3- bis 15-gliedrigen gesättigten oder partiell ungesättigten Heterocyclyloxy, $C_6$-$C_{14}$-Aryl, $C_6$-$C_{14}$-Aryloxy, 5- bis 14-gliedriges Heteroaryl und 5- bis 14-gliedriges Heteroaryloxy, wobei die acht letztgenannten Reste unsubstituiert sind oder einen oder mehrere Reste $R^{1a}$ tragen, wobei

jedes $R^{1a}$ unabhängig voneinander für $NR^{1b}R^{1c}$, $C_1$-$C_{20}$-Alkyl, $C_2$-$C_{20}$-Alkenyl, $C_2$-$C_{20}$-Alkinyl, $C_1$-$C_{20}$-Alkoxy, $C_1$-$C_{20}$-Alkoxy-$C_1$-$C_{20}$-alkyl, $C_1$-$C_{20}$-Alkoxy, das im Alkylteil ein- oder mehrfach durch nicht benachbarte Sauerstoffatome unterbrochen ist, $C_1$-$C_{20}$-Alkylthio, $C_1$-$C_{20}$-Haloalkyl, $C_1$-$C_{20}$-Haloalkoxy, $C_2$-$C_{20}$-Haloalkenyl, $C_2$-$C_{20}$-Haloalkinyl, $C_2$-$C_{20}$-Alkenyloxy, $C_2$-$C_{20}$-Alkinyl-oxy, $C_6$-$C_{14}$-Aryl-$C_1$-$C_{10}$-alkyl, $C_6$-$C_{14}$-Aryl-$C_2$-$C_{10}$-alkenyl oder $C_6$-$C_{14}$-Aryl-$C_2$-$C_{10}$-alkinyl steht,

wobei

$R^{1b}$ und $R^{1c}$ unabhängig voneinander für $C_1$-$C_{20}$-Alkyl oder $C_6$-$C_{14}$-Aryl stehen;

wobei die aliphatischen oder aromatischen Gruppen in der vorgenannten Gruppe $R^{1a}$ ihrerseits eine, zwei oder drei Gruppen $NR^{1b}R^{1c}$ tragen können,

einer der Reste $R^3$ oder $R^4$ für 5- bis 14-gliedriges Heteroaryl steht, das unsubstituiert ist oder einen oder mehrere Reste $R^x$ trägt, und der andere Rest $R^3$ oder $R^4$ für eine elektronenziehende Gruppe $E^1$ steht,

wobei

jedes $R^x$ unabhängig voneinander für Halogen, $C_1$-$C_{20}$-Alkyl, $C_2$-$C_{20}$-Alkenyl, $C_2$-$C_{20}$-Alkinyl, $C_1$-$C_{20}$-Alkoxy, $C_1$-$C_{20}$-Alkylthio, $C_1$-$C_{20}$-Haloalkyl, $C_1$-$C_{20}$-Haloalkoxy, $C_2$-$C_{20}$-Haloalkenyl, $C_2$-$C_{20}$-Haloalkinyl, $C_2$-$C_{20}$-Alkenyloxy, $C_2$-$C_{20}$-Alkinyloxy, $C_6$-$C_{14}$-Aryl, $C_6$-$C_{14}$-Aryl-$C_1$-$C_{10}$-alkyl, $C_6$-$C_{14}$-Aryl-$C_2$-$C_{10}$-alkenyl oder $C_6$-$C_{14}$-Aryl-$C_2$-$C_{10}$-alkinyl steht; und

$E^1$ für Cyano, Nitro, $SO_3H$, CHO, $COR^{E1}$, $COOR^{E1}$, $N(R^{E2})_3{}^+X^-$ steht, wobei $R^{E1}$ für Wasserstoff oder $C_1$-$C_{10}$-Alkyl steht; jedes $R^{E2}$ unabhängig voneinander für Wasserstoff oder $C_1$-$C_{10}$-Alkyl steht; und $X^-$ für ein Anionäquivalent steht;

einer der Reste $R^5$ oder $R^6$ für 5- bis 14-gliedriges Heteroaryl steht, das unsubstituiert ist oder einen oder mehrere Reste $R^x$ trägt, und der andere Rest $R^5$ oder $R^6$ für eine elektronenziehende Gruppe $E^2$ steht, wobei $E^2$ wie $E^1$ definiert ist;

$R^7$ und $R^8$, unabhängig voneinander, ausgewählt sind unter Wasserstoff, $C_1$-$C_{20}$-Alkyl und $B(R^9R^{10})$, wobei

$R^9$ für Halogen, $C_1$-$C_{10}$-Alkyl, $C_3$-$C_{10}$-Cycloalkyl, $C_3$-$C_{15}$-Cycloalkenyl oder $C_6$-$C_{14}$-Aryl steht, wobei die drei letztgenannten zyklischen Reste unsubstituiert sind oder einen oder mehrere Reste $R^{9a}$ tragen, wobei $R^{9a}$ wie $R^x$ definiert ist; und

$R^{10}$ wie $R^9$ definiert ist.

Gegenstand der vorliegenden Erfindung ist weiterhin eine organische Solarzelle mit einem photoaktiven Bereich, der wenigstens ein organisches Donormaterial in Kontakt mit wenigstens einem organischen Akzeptormaterial aufweist, wobei das Donormaterial und das Akzeptormaterial einen Donor-Akzeptor-Heteroübergang ausbilden und wobei der photoaktive Bereich wenigstens eine Verbindung der Formel I, wie zuvor definiert, enthält.

Gegenstand der vorliegenden Erfindung ist weiterhin ein organischer Feldeffekttransistor, enthaltend ein Substrat mit wenigstens einer Gate-Struktur und einer Drain- und Sourceelektrode und wenigstens eine Verbindung der Formel I, wie zuvor definiert.

Gegenstand der vorliegenden Erfindung ist weiterhin ein Substrat mit einer Vielzahl von organischen Feldeffekttransistoren, wobei zumindest ein Teil der Feldeffekttransistoren wenigstens eine Verbindung der Formel I, wie zuvor definiert, enthält; ein Halbleiterbaustein, umfassend wenigstens ein Substrat, wie zuvor definiert, sowie eine organische Leuchtdiode enthaltend mindestens eine Verbindung der allgemeinen Formel I, wie zuvor definiert.

Für die in den vorgenannten erfindungsgemäßen Vorrichtungen wird die Verwendung der Verbindung der Formel I,

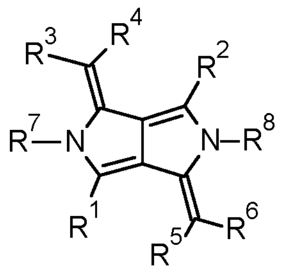

(I)

worin

R1 für 4-Methoxyphenyl steht;
R2 für 4-Methoxyphenyl steht;
R3 für 6-tert-Butyl-chinolin-2-yl steht;
R4 für Cyano steht;
R5 für Cyano steht;
R6 für 6-tert-Butyl-chinolin-2-yl steht;
R7 für BF2 steht; und
R8 für BF2 steht, vorgeschlagen.

Bei den in den vorstehenden Formeln angegebenen Definitionen der Variablen werden Sammelbegriffe verwendet, die allgemein repräsentativ für die jeweiligen Substituenten stehen. Die Bedeutung $C_n$-$C_m$ gibt die jeweils mögliche Anzahl von Kohlenstoffatomen in dem jeweiligen Substituenten oder Substituentenanteil an:
Halogen: Fluor, Chlor, Brom oder Iod.

Alkyl sowie Alkylteile in Alkoxy und Alkylthio: gesättigte, geradkettige oder verzweigte Kohlenwasserstoffreste mit 1 bis 20 ($C_1$-$C_{20}$-Alkyl), häufig 1 bis 10 ($C_1$-$C_{10}$-Alkyl) Kohlenstoffatomen wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl, Heptyl, 1-Methylhexyl, Octyl, 1-Methylheptyl, 2-Ethylhexyl, n-Nonyl, n-Decyl.

Unter Alkyl, welches durch ein oder mehrere, beispielsweise ein oder zwei, nicht benachbarte Sauerstoffatome unterbrochen ist, ist beispielsweise 2-Methoxyethyl, 2- und 3-Methoxy-propyl, 2-Ethoxyethyl, 2- und 3-Ethoxypropyl, 2-Propoxyethyl, 2- und 3-Propoxypropyl, 2-Butoxyethyl, 2- und 3-Butoxypropyl, 3,6-Dioxaheptyl und 3,6-Dioxaoctyl zu nennen.

Halo(gen)alkyl sowie alle Halo(gen)alkylteile in Halo(gen)alkoxy: geradkettige oder verzweigte Alkylgruppen mit 1 bis 20 und insbesondere 1 bis 10 Kohlenstoffatomen (wie vorstehend genannt), wobei in diesen Gruppen teilweise oder vollständig die Wasserstoffatome durch Halogenatome wie vorstehend genannt, ersetzt sind;
Alkenyl: einfach ungesättigte, geradkettige oder verzweigte Kohlenwasserstoffreste mit 2 bis 20 ($C_2$-$C_{20}$-Alkenyl) oder 3 bis 10 ($C_3$-$C_{10}$-Alkenyl) Kohlenstoffatomen und einer Doppelbindung in einer beliebigen Position, z. B. Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl;
Alkinyl: geradkettige oder verzweigte Kohlenwasserstoffgruppen mit 2 bis 20 ($C_2$-$C_{20}$-Alkinyl) oder 3 bis 10 ($C_3$-$C_{10}$-Alkinyl) Kohlenstoffatomen und einer Dreifachbindung in einer beliebigen Position, z. B. Ethinyl, 1-Propinyl, 2-Propinyl, 1-Butinyl, 2-Butinyl, 3-Butinyl, 1-Methyl-2-propinyl;
Cycloalkyl: mono- oder bicyclische, gesättigte Kohlenwasserstoffgruppen mit 3 bis 6, 8, 10 oder 15 Kohlenstoffringgliedern, z. B. monocyclisches $C_3$-$C_8$-Cycloalkyl wie Cyclopropyl, Cyclobutyl, Cyclopentyl, Cyclohexyl, Cycloheptyl oder Cyclooctyl, bicyclisches $C_6$-$C_{15}$-Cycloalkyl wie Bicyclo[2.2.1]hept-1-yl, Bicyclo[2.2.1]hept-2-yl, Bicyclo[2.2.1]-hept-7-yl, Bicyclo[2.2.2]oct-1-yl, Bicyclo[2.2.2]oct-2-yl, Bicyclo[3.3.0]octyl und Bicyclo-[4.4.0]decyl;
$C_3$-$C_{15}$-Cycloalkyloxy ($C_3$-$C_{15}$-Cycloalkoxy): $C_3$-$C_{15}$-Cycloalkyl, wie zuvor definiert, welches über ein Sauerstoffatom

(-O-) an das Gerüst gebunden ist;
Cycloalkenyl: monocyclische, einfach ungesättigte Kohlenwasserstoffgruppen mit 3 bis 6, 8, 10 oder 15, vorzugsweise 5 bis 8, Kohlenstoffringgliedern, wie Cyclopenten-1-yl, Cyclopenten-3-yl, Cyclhexen-1-yl, Cyclohexen-3-yl und Cyclohexen-4-yl;
Aryl: ein-, zwei- oder dreikernige (monocyclische, bicyclische oder tricyclische) aromatische Kohlenwasserstoffreste mit 6 bis 14, besonders bevorzugt 6 bis 10 Kohlenstoffatomen, die keine Ringheteroatome enthalten. Beispiele für Aryl sind insbesondere Phenyl, Naphthyl, Indenyl, Fluorenyl, Anthracenyl, Phenanthrenyl, und speziell Phenyl oder Naphthyl;
$C_6$-$C_{14}$-Aryloxy: $C_6$-$C_{14}$-Aryl, wie zuvor definiert, welches über ein Sauerstoffatom (-O-) an das Gerüst gebunden ist. Bevorzugt sind Phenoxy und Naphthyloxy.

3- bis 15-gliedriger gesättigter oder partiell ungesättigter Heterocyclus: ein-, zwei- oder dreikerniges gesättigtes oder partiell ungesättigtes Ringsystem mit 3, 4, 5, 6, 7, 8, 9, 10, 11, 12, 13, 14 oder 15 Ringgliedern, enthaltend neben Kohlenstoffatomen als Ringglieder ein, zwei, drei oder vier Heteroatome oder heteroatomhaltige Gruppen, ausgewählt unter O, N, S, SO und $S(O)_2$ als Ringglieder;
3- bis 15-gliedriges gesättigtes oder partiell ungesättigtes Heterocyclyloxy: 3- bis 15-gliedriges gesättigter oder, partiell ungesättigter Heterocyclus wie zuvor definiert, welcher über ein Sauerstoffatom (-O-) an das Gerüst gebunden ist.

5- bis 14-gliedriges Het(ero)aryl: ein-, zwei- oder dreikerniges (monozyklisches, bizyklisches oder trizyklisches) aromatisches Ringsystem mit 5 bis 14 Ringgliedern, die sich zum Teil von vorstehend genannten Aryl ableiten lassen, in dem im Aryl-Grundgerüst mindestens ein Kohlenstoffatom durch ein Heteroatom ersetzt ist. Bevorzugte Heteroatome sind N, O und S. Besonders bevorzugt weisen die Heteroarylreste 5 bis 13 Ringatome auf. Besonders bevorzugt weisen die Heteroarylreste neben Kohlenstoffatomen ein, zwei, drei oder vier unter O, S und N ausgewählte Heteroatome als Ringglieder auf. Insbesondere bevorzugt ist das Grundgerüst der Heteroarylreste ausgewählt aus Systemen wie:

- fünf- oder sechsgliedriger aromatischer Heterocyclus, enthaltend ein, zwei, drei oder vier Heteroatome aus der Gruppe Sauerstoff, Stickstoff oder Schwefel: z. B. C-gebundenes 5-gliedriges Heteroaryl, enthaltend ein bis drei Stickstoffatome oder ein oder zwei Stickstoffatome und/oder ein Schwefel- oder Sauerstoffatom als Ringglieder wie 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol-2-yl, 1,3,4-Thiadiazol-2-yl und 1,3,4-Triazol-2-yl; über Stickstoff gebundenes 5-gliedriges Heteroaryl, enthaltend ein bis drei Stickstoffatome als Ringglieder, wie Pyrrol-1-yl, Pyrazol-1-yl, Imidazol-1-yl, 1,2,3-Triazol-1-yl und 1,2,4-Triazol-1-yl; 6-gliedriges Heteroaryl, enthaltend ein bis drei Stickstoffatome als Ringglieder wie Pyridin-2-yl, Pyridin-3-yl, Pyridin-4-yl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl und 1,2,4-Triazin-3-yl;
- benzokondensierter fünf- oder sechsgliedriger aromatischer Heterocyclus, enthaltend ein, zwei, drei oder vier, vorzugsweise ein, zwei oder drei Heteroatome aus der Gruppe Sauerstoff, Stickstoff oder Schwefel: z. B. fünf- oder sechsgliedrige aromatische Heterocyclen, wie zuvor definiert, welche neben Kohlenstoffatomen ein bis vier Stickstoffatome oder ein bis drei Stickstoffatome und ein Schwefel- oder Sauerstoffatom als Ringglieder enthalten können, und in welchen zwei benachbarte Kohlenstoffringglieder oder ein Stickstoff- und ein benachbartes Kohlenstoffringglied durch eine Buta-1,3-dien-1,4-diylgruppe verbrückt sein können wie Indolyl, Indazolyl, Benzofuryl, Dibenzofuryl, Isobenzofuranyl, Benzothiophenyl, Dibenzothiophenyl, Benzothiazolyl, Benzoxazolyl, Benzimidazolyl, Carbazolyl, Chinolinyl, Isochinolinyl, Chinazolinyl, Chinoxalinyl, Cinnolinyl, Phthalazinyl, Purinyl Acridinyl, Phenanthridinyl, Phenazinyl und 1,7-Phenanthrolinyl.

X- steht für ein Anionäquivalent, d. h. für ein einwertiges Anion oder den einer negativen Einfachladung entsprechenden Anteil eines mehrwertigen Anions. Das Anion $X^-$ dient lediglich als Gegenion zur Neutralisation der positiv geladenen Substituentengruppe $N(R^{E2})_3{}^+$ und kann im Prinzip beliebig gewählt werden, wobei im Allgemeinen Halogenid-Ionen X- bevorzugt sind.

Ein organisches Material wird im Sinne der vorliegenden Anmeldung als löcherleitend bezeichnet, wenn in dem Material die Ladungsträger, die in Folge von Lichtabsoprtion und Ladungstrennung an einem Heteroübergang gebildeten werden ("photogenerierte Ladungsträger"), in Form von Löchern transportiert werden. In analoger Weise wird ein organisches Material als elektronenleitend bezeichnet, wenn in dem Material photogenerierte Ladungsträger in Form von Elektronen transportiert werden. Ein Grenzflächenbereich zwischen dem elektronenleitenden und dem löcherleitenden Material wird als Heteroübergang bezeichnet.

Gemäß einer bevorzugten Ausführungsform der vorliegenden Erfindung ist die Vorrichtung ausgewählt unter einer organischen Solarzelle, einer organischen Elektrolumineszenzvorrichtung, einem Photodetektor und einem organischen Feldeffekttransistor. Insbesondere ist die Vorrichtung eine organische Solarzelle.

Hinsichtlich des Einsatzes der Verbindung der Formel I in einer Vorrichtung der organischen Elektronik weisen die Variablen $R^1$, $R^2$, $R^3$, $R^4$, $R^5$, $R^6$, $R^7$ und $R^8$ unabhängig voneinander und bevorzugt in Kombination vorzugsweise

die im Folgenden angegebenen Bedeutungen auf:
Bevorzugt sind Verbindungen der Formel I, in denen $R^1$ und $R^2$ unabhängig voneinander ausgewählt sind unter $C_6$-$C_{10}$-Aryl, das unsubstituiert ist oder 1, 2 oder 3 Reste $R^{1a}$ trägt und 5- bis 14-gliedrigem Heteroaryl, das unsubstituiert ist oder 1, 2 oder 3 Reste $R^{1a}$ trägt, wobei Heteroaryl 1, 2 oder 3 unter N, O und S ausgewählte Heteroatome als Ringglieder enthält. Wenn Aryl oder Heteroaryl eine, zwei oder drei, gleiche oder verschiedene Gruppen $R^{1a}$ trägt, so ist $R^{1a}$ vorzugsweise ausgewählt unter $C_1$-$C_6$-Alkyl, $C_2$-$C_8$-Alkenyloxy, $C_1$-$C_{10}$-Alkoxy, Di-($C_1$-$C_{10}$-alkyl)amino und Di-($C_1$-$C_4$-Alkyl)amino-$C_1$-$C_8$-alkoxy. Spezielle Beispiele für $R^{1a}$ sind Methoxy, 4-Butyloxy, 4-Octyloxy, Hex-5-enyloxy und N-Methyl-N-octylamino. Vorzugsweise trägt Aryl oder Heteroaryl eine Gruppe $R^{1a}$. Insbesondere werden Verbindungen I bevorzugt, in denen $R^1$ und $R^2$ die gleiche Bedeutung aufweisen. Insbesondere stehen $R^1$ und $R^2$ jeweils für Phenyl, das eine Gruppe $R^{1a}$ trägt. In dieser Ausführungsform weist $R^{1a}$ vorzugsweise eine der als bevorzugt genannten Bedeutungen auf.

Außerdem werden Verbindungen der Formel I bevorzugt, worin einer der Reste $R^3$ oder $R^4$ und einer der Reste $R^5$ oder $R^6$ unabhängig voneinander ausgewählt sind unter 5- oder 6-gliedrigem monozyklischen Heteroaryl und 9- oder 10-gliedrigem bizyklischen Heteroaryl, wobei 5- oder 6-gliedriges monozyklisches Heteroaryl und 9- oder 10- gliedriges bizyklisches Heteroaryl unsubstituiert sind oder einen oder mehrere Reste $R^x$ aufweisen. 5- oder 6-gliedriges monozyklisches Heteroaryl und 9- oder 10-gliedriges bizyklisches Heteroaryl enthalten als Ringglieder neben Kohlenstoffatomen 1, 2, 3 oder 4 unter O, S und N ausgewählte Heteroatome. Vorzugsweise ist wenigstens ein Ringatom (Ringglied) ein Stickstoffatom. Besonders bevorzugt ist wenigstens ein Stickstoffringatom in alpha-Position zu der Verknüpfungsstelle mit dem Pyrrolopyrrol-Gerüst angeordnet. Wenn 5- oder 6-gliedriges monozyklisches Heteroaryl und 9- oder 10-gliedriges bizyklisches Heteroaryl eine, zwei, drei, vier oder fünf gleiche oder verschiedene Gruppen $R^x$ trägt, so ist $R^x$ vorzugsweise Brom, Chlor, Phenyl, $C_1$-$C_6$-Alkyl oder $C_1$-$C_6$-Haloalkyl, insbesondere lineares oder verzweigtes $C_1$-$C_4$-Alkyl oder lineares oder verzweigtes $C_1$-$C_4$-Haloalkyl. Insbesondere tragen 5- oder 6-gliedriges monozyklisches Heteroaryl und 9- oder 10-gliedriges bizyklisches Heteroaryl eine, zwei oder drei, insbesondere eine oder zwei Gruppen $R^x$.

Bevorzugte erfindungsgemäß verwendete Verbindungen der Formel I zeichnen sich dadurch aus, dass einer der Reste $R^3$ oder $R^4$ und einer der Reste $R^5$ oder $R^6$ unabhängig voneinander ausgewählt sind unter Resten der Formeln Het-1, Het-2, Het-3, Het-4, Het-5 und Het-6

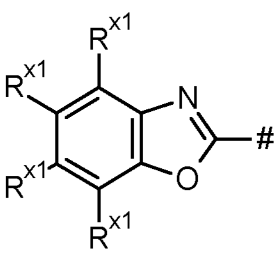

Het-1

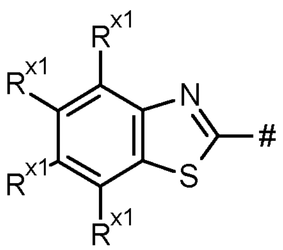

Het-2

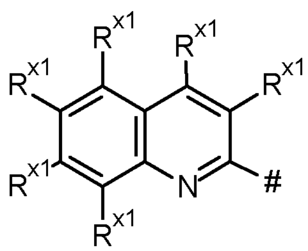

Het-3

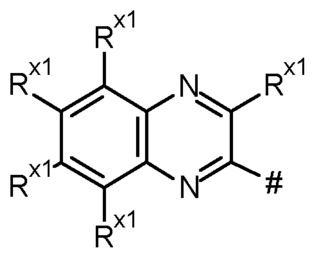

Het-4

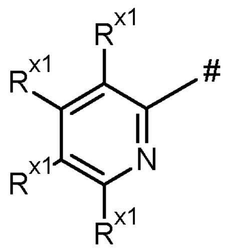

Het-5

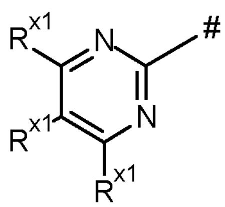

Het-6

worin

# für die Bindungsstelle zu dem Pyrrolopyrrol-Grundgerüst steht; und
jedes $R^{x1}$ unabhängig voneinander für Wasserstoff steht oder eine der für $R^x$ genannten Bedeutungen aufweist.

Ganz besonders bevorzugt sind Verbindungen der Formel I, worin einer der Reste $R^3$ oder $R^4$ und einer der Reste $R^5$ oder $R^6$ unabhängig voneinander für 5-tert-Butyl-1,3-benzoxazol-2-yl, 6-tert-Butyl-1,3-benzoxazol-2-yl, 5-tert-Butyl-1,3-benzthiazol-2-yl, 6-tert-Butyl-1,3-benzthiazol-2-yl, 6-Chinoxalin-2-yl, 6-tert-Butylchinolin-2-yl, 6-Methylpyridin-2-yl oder 4,6-Dimethylpyrimidin-2-yl stehen.

Wenn einer der Reste $R^3$ oder $R^4$ für gegebenenfalls substituiertes 5- bis 14-gliedriges Heteroaryl steht, so steht der andere Rest $R^3$ oder $R^4$ für eine elektronenziehende Gruppe $E^1$. Wenn einer der Reste $R^5$ oder $R^6$ für gegebenenfalls substituiertes Heteroaryl steht, so steht der andere Rest $R^5$ oder $R^6$ für eine elektronenziehende Gruppe $E^2$. Bevorzugt ist $E^1$ ausgewählt unter Nitro und Cyano, insbesondere Cyano. Bevorzugt ist $E^2$ ausgewählt unter Nitro und Cyano, insbesondere Cyano. In einer bevorzugten Ausführungsform weisen $E^1$ und $E^2$ die gleiche Bedeutung auf. Ganz besonders bevorzugt stehen $E^1$ und $E^2$ für Cyano.

In einer weiteren bevorzugten Ausführungsform sind $R^3$ und $R^6$ jeweils ausgewählt unter gegebenenfalls substituiertem 5- oder 6-gliedrigen monozyklischen Heteroaryl und 9- oder 10-gliedrigem gegebenenfalls substituiertem bizyklischen Heteroaryl und $R^3$ und $R^6$ weisen die gleiche Bedeutung auf. In einer weiteren speziellen Ausführungsform sind $R^3$ und $R^6$ nicht identisch.

In einer weiteren besonders bevorzugten Ausführungsform weisen in der Verbindung der Formel I die Reste $R^3$ und $R^6$ und die Reste $R^4$ und $R^5$ die gleiche Bedeutung auf.

In einer weiteren besonders bevorzugten Ausführungsform weisen in der Verbindung der Formel I die Reste $R^4$ und $R^5$ die gleiche Bedeutung auf und $R^3$ und $R^6$ weisen unterschiedliche Bedeutungen auf.

In einer weiteren speziellen Ausführungsform sind $R^7$ und $R^8$ unabhängig voneinander ausgewählt unter Wasserstoff, $C_1$-$C_4$-Alkyl, $BF_2$, $B(C_1\text{-}C_8\text{-Alkyl})_2$, $B(\text{Phenyl})_2$ und $B(\text{Phenyl})_2$, worin Phenyl ein, zwei, drei, vier oder fünf Substituenten trägt. Die Substituenten am Phenylring sind vorzugsweise unabhängig voneinander unter $C_1$-$C_4$-Alkyl, $C_2$-$C_{10}$-Alkenyl und $C_1$-$C_4$-Alkoxy ausgewählt. In einer bevorzugten Ausführungsform weisen $R^7$ und $R^8$ die gleiche Bedeutung auf. Gemäß dieser Ausführungsform sind $R^7$ und $R^8$ ausgewählt unter Wasserstoff, $BF_2$, $B(C_1\text{-}C_8\text{-Alkyl})_2$ und $B(\text{Phenyl})_2$. In einer weiteren Ausführungsform weisen $R^7$ und $R^8$ unterschiedliche Bedeutungen auf.

In einer besonders bevorzugten Ausführungsform weisen die Variablen $R^1$, $R^2$, $R^3$, $R^4$, $R^5$, $R^6$, $R^7$ und $R^8$ unabhängig voneinander und bevorzugt in Kombination vorzugsweise die im Folgenden angegebenen Bedeutungen auf:

> $R^1$ und $R^2$ sind identisch und ausgewählt unter Phenyl und Phenyl, das einen Substituenten $R^{1x}$ trägt. $R^{1x}$ ist wie zuvor definiert und weist vorzugsweise eine bevorzugte Bedeutung auf, insbesondere $C_1$-$C_{10}$-Alkoxy;
> $R^3$ und $R^6$ stehen jeweils für Cyano;
> $R^4$ und $R^5$ stehen, unabhängig voneinander für einen Rest ausgewählt unter Het-1, Het-2, Het-3, Het-4, Het-5 und Het-6; und
> $R^7$ und $R^8$ sind unabhängig voneinander ausgewählt unter Wasserstoff, $BF_2$ und $B(\text{Phenyl})_2$.

In einer ganz besonders bevorzugten Ausführungsform weisen die Variablen $R^1$, $R^2$, $R^3$, $R^4$, $R^5$, $R^6$, $R^7$ und $R^8$ unabhängig voneinander und bevorzugt in Kombination vorzugsweise die im Folgenden angegebenen Bedeutungen auf:

> $R^1$ und $R^2$ sind identisch und ausgewählt unter Phenyl und Phenyl, das einen Substituenten $R^{1x}$ trägt. $R^{1x}$ ist wie zuvor definiert und weist vorzugsweise eine der bevorzugten Bedeutungen auf, insbesondere $C_1$-$C_{10}$-Alkoxy;
> $R^3$ und $R^6$ stehen jeweils für Cyano;
> $R^4$ und $R^5$ sind identisch und ausgewählt unter 5-tert-Butyl-1,3-benzoxazol-2-yl, 6-tert-Butyl-1,3-benzoxazol-2-yl, 5-tert-Butyl-1,3-benzthicizol-2-yl, 6-tert-Butyl-1,3-benzthiazol-2-yl, 6-Chinoxalin-2-yl, 6-tert-Butylchinolin-2-yl, 6-Methylpyridin-2-yl und 4,6-Dimethylpyrimidin-2-yl; und
> $R^7$ und $R^8$ sind unabhängig voneinander ausgewählt unter Wasserstoff, $BF_2$ und $B(\text{Phenyl})_2$.

Die Verbindung der Formel I, worin

$R^1$ für 4-Methoxyphenyl steht;
$R^2$ für 4-Methoxyphenyl steht;
$R^3$ für 6-tert-Butyl-chinolin-2-yl steht;
$R^4$ für Cyano steht;
$R^5$ für Cyano steht; und
$R^6$ für 6-tert-Butyl-chinolin-2-yl steht;
$R^7$ für $BF_2$ und
$R^8$ für $BF_2$ steht

ist neu und für die Verwendung in den erfindungsgemäßen Vorrichtungen geeignet.

Die Herstellung der in der erfindungsgemäßen Vorrichtung eingesetzten Pyrrolopyrrol-Cyanin-Verbindungen der Formel I kann nach üblichen, dem Fachmann bekannten Verfahren erfolgen.

Die Herstellung der Verbindungen I, worin $R^7$ und $R^8$ jeweils für Wasserstoff stehen, erfolgt in der Regel durch Kondensationsreaktion einer Diketopyrrolopyrrol-Verbindung der allgemeinen Formel II

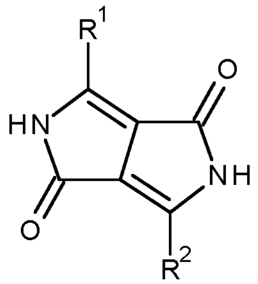

(II)

mit einer durch eine elektronenziehenden Gruppe aktivierten Verbindung der allgemeinen Formel $CH_2R^3R^4$ und gegebenenfalls einer durch eine elektronenziehende Gruppe aktivierten Verbindung der Formel $CH_2R^5R^6$, die von der Verbindung der allgemeinen Formel $CH_2R^3R^4$ verschieden ist.

Kondensationsreaktionen von Diketopyrrolopyrrolen sind prinzipiell bekannt. Die Umsetzung erfolgt üblicherweise in Gegenwart einer Verbindung, die sich zur Aktivierung von Carbonylgruppen eignet. Ein Beispiel für ein geeignetes Aktivierungsmittel ist $POCl_3$. Das molare Verhältnis von Diketoverbindung der Formel II zu beispielsweise $POCl_3$ beträgt üblicherweise 1: 2 bis 1: 12, vorzugsweise 1:4 bis 1:10. Vorzugsweise erfolgt die Umsetzung der Diketoverbindung mit der durch eine elektronenziehende Gruppe aktivierten Verbindung der Formel $CH_2R^3R^4$ in Gegenwart eines hochsiedenden Lösungsmittels.

Als Lösungsmittel für die Kondensationsreaktion eignen sich unpolar aprotische Lösungsmittel, wie Kohlenwasserstoffe, z. B. Benzol, Toluol, Xylol, Mesitylen, Petrolether, Dekalin und Ether. Die Reaktionstemperatur hängt von der Reaktivität der Edukte ab und liegt im Allgemeinen im Bereich von 50 bis 250 °C.

Die Umsetzung erfolgt üblicherweise unter Schutzgasatmosphäre.

Im Fall, dass die durch eine elektronenziehende Gruppe aktivierte Verbindung der allgemeinen Formel $CH_2R^3R^4$ die gleiche Bedeutung aufweist wie die durch eine elektronenziehende Gruppe aktivierte Verbindung der allgemeinen Formel $CH_2R^5R^6$, beträgt das Molverhältnis von Diketopyrrolopyrrol-Verbindung II zu der Verbindung der allgemeinen Formel $CH_2R^3R^4$ in der Regel 1 : 2 bis 1: 4.

Im Fall, dass die durch eine elektronenziehende Gruppe aktivierte Verbindung der allgemeinen Formel $CH_2R^3R^4$ von der durch eine elektronenziehende Gruppe aktivierte Verbindung der allgemeinen Formel $CH_2R^5R^6$ verschieden ist, setzt man zunächst die Verbindung der Formel II mit der Verbindung der Formel $CH_2R^3R^4$ unter Erhalt der Verbindung III und anschließend die Verbindung der Formel III mit der Verbindung der Formel $CH_2R^5R^6$ um gemäß der in Schema 1 dargestellten Methode.

Schema 1:

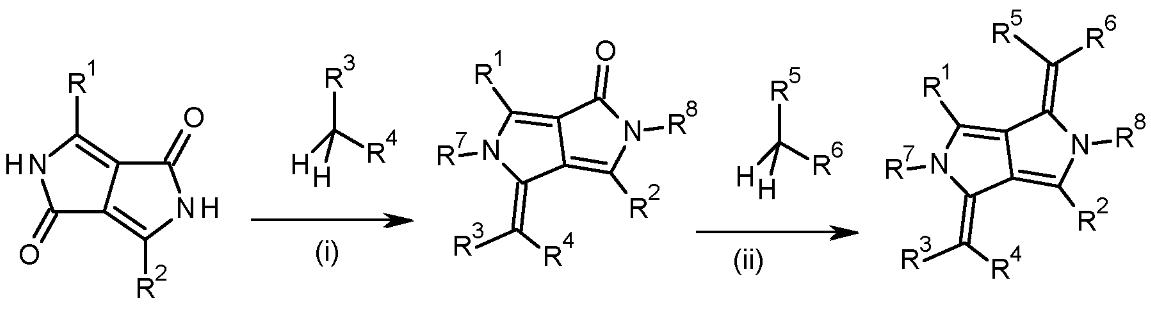


(II) (III) (I) $R^7 = R^8 = H$

In Schema 1 haben $R^1$, $R^2$, $R^3$, $R^4$, $R^5$ und $R^6$ die zuvor genannten Bedeutungen. Sowohl die Umsetzung in Schritt (i) des Schemas 1 als auch die Umsetzung in Schritt (ii) des Schemas 1 erfolgen in Gegenwart von $POCl_3$. Die Reaktionstemperatur in Schritt (i) ist in der Regel niedriger als in Schritt (ii). Geeignete Lösungsmittel für Schritt (i) sind die zuvor genannten unpolar aprotischen Lösungsmittel. Geeignete Ether sind cyclische Ether, z. B. Tetrahydrofuran.

Verbindungen der Formel I, worin $R^7$ und $R^8$ für $C_1$-$C_4$-Alkyl stehen, lassen sich aus Verbindungen der Formel I, worin $R^7$ und $R^8$ jeweils für Wasserstoff stehen durch Umsetzung mit einem Alkylierungsmittel der Formel ($C_1$-$C_4$-Al-

kyl)-LG herstellen. LG steht für eine nucleophil verdrängbare Abgangsgruppe. Beispiele für eine geeignete, nucleophil verdrängbare Abgangsgruppe LG sind Halogenid, vorzugsweise Chlorid, Bromid oder Iodid, Sulfat, $C_1$-$C_{18}$-Alkysulfonyloxy, $C_1$-$C_{18}$-Halogenalkylsulfonyloxy, $C_1$-$C_{18}$-Alkoxysulfonyloxy, oder Phenylsulfonyloxy, worin der Phenylrest gegebenenfalls mit Halogen, Nitro oder $C_1$-$C_6$-Alkyl ein- oder mehrfach substituiert ist wie Phenylsulfonyloxy, p-Toluolsulfonyloxy, p-Chlorphenylsulfonyloxy, p-Bromphenylsulfonyloxy oder p-Nitrophenylsulfonyloxy.

Üblicherweise führt man die Alkylierung in Gegenwart einer Base durch. Geeignete Basen sind z. B. Alkali- und Erdalkalimetallcarbonate wie Lithiumcarbonat, Natriumcarbonat, Kaliumcarbonat oder Cäsiumcarbonat oder Alkali- und Erdalkalimetallhydroxide wie Natriumhydroxid, Kaliumhydroxid und Lithiumhydroxid.

Verbindungen der Formel I, worin $R^7$ und $R^8$ für $B(R^9R^{10})$ stehen, lassen sich aus Verbindungen der Formel I, worin $R^7$ und $R^8$ jeweils für Wasserstoff stehen, durch Umsetzung mit Verbindungen der Formel $B(R^9)(R^{10})Hal$, worin Hal für Halogen wie Brom, Chlor oder Fluor steht, herstellen. Geeignete Verbindungen der Formel $B(R^9)(R^{10})Hal$ sind beispielsweise Bortrifluorid-Diethylether, Bortrichlorid-Diethylether, Diphenylborchlorid oder Di($C_1$-$C_4$-alkyl)borchlord. Die Umsetzung erfolgt üblicherweise in Gegenwart einer Base wie Diisopropylethylamin (Hünig Base). Die Umsetzung erfolgt üblicherweise in einem Lösungsmittel. Geeignete Lösungsmittel sind chlorierte Benzole wie 1,2-Dichlorbenzol oder chlorierte Kohlenwasserstoffe wie Dichlormethan.

Die Herstellung von Verbindungen der Formel I, worin $E^1$ und $E^2$ für Cyano stehen, ist beispielsweise aus Angew. Chem., 119 (2007) 3824-3827 (Angew. Chem. Int. Ed. 46 (2007) 3750-3753), Angew. Chem. 2011, 123, 1442-1445 (Angew. Chem. Int. Ed., 50 (2011) 1406-1409), Eur. J. Org. Chem. 2011, 3421-3429, Chem. Comm., 46 (2010), 5289-5291 und Chem. Eur. J., 15 (2009) 4857-4864 bekannt.

Verbindungen der Formel II sind bekannt oder können nach literaturbekannten Verfahren hergestellt werden, z. B. gemäß Chem. Eur. J., 15 (2009) 4857-4864 oder Angew. Chem., 119 (2007) 3824-3827 (Angew. Chem. Int. Ed. 46 (2007) 3750-3753).

Verbindungen der Formel $CH_2R^3R^4$ und $CH_2R^5R^6$ sind bekannt oder können nach literaturbekannten Verfahren hergestellt werden beispielsweise wie in Chem. Eur. J., 15 (2009) 4857-4864 beschrieben.

Die in den erfindungsgemäßen Vorrichtungen eingesetzten Pyrrolopyrrol-Cyanine der allgemeinen Formel I zeichnen sich durch wenigstens eine der folgenden vorteilhaften Eigenschaften aus:

- sehr gute Zugänglichkeit durch einfache Syntheseverfahren,
- hohe thermische Stabilität,
- gute Sublimierbarkeit,
- hohe Fotostabilität,
- ein Hauptabsorptionsmaximum im NIR-Bereich von 650 nm bis 1000 nm,
- sehr hohe Absorption mit schmaler Linienbreite im NIR-Bereich,
- schwache oder fehlende Absorption im sichtbaren Bereich,
- sehr gute Ladungstransporteigenschaften,
- lange Lebensdauer der daraus hergestellten elektronischen Bauelemente, speziell Solarzelle.

Verbindungen der Formel I, worin $R^7$ oder $R^8$ oder $R^7$ und $R^8$ für einen Rest $B(R^9\ R^{10})$ stehen, zeigen - im Gegensatz zu den Verbindungen der allgemeinen Formel I, in denen $R^7$ und $R^8$ für Wasserstoff stehen, - keine große innermolekulare Beweglichkeit mehr. Verbindungen der Formel I, worin $R^7$ und $R^8$ für einen Rest $B(R^9\ R^{10})$ stehen, zeigen eine scharfe Absorptionsbande im Spektralbereich zwischen 650 und 900 nm, sehr geringe Absorption im sichtbaren Bereich und hohe Fluoreszenzquantenausbeuten. Verbindungen der Formel I, worin $R^7$ und $R^8$ für einen Rest $B(R^9R^{10})$ stehen, zeigen im Vergleich zu Verbindungen der Formel I, in denen $R^7$ und $R^8$ für Wasserstoff stehen, eine höhere optische Dichte. Die optische Dichte ist eine andere Bezeichnung für die von der Wellenlänge abhängige Extinktion $E_\lambda$. Sie ist ein Maß für die Abschwächung der Strahlung nach Durchgang durch ein Medium.

Die Pyrrolopyrrol-Cyanin-Verbindungen der allgemeinen Formel I eignen sich vorteilhaft zur Verwendung in elektronischen Bauelementen. Dazu zählen vor allem organische Leuchtdioden (organische lichtemittierende Dioden, organic light emitting diods, OLED), organische Feldeffekttransistoren (Organic Field-Effect Transistors, OFET) sowie die organische Photovoltaik.

Vor der Verwendung in einer erfindungsgemäßen Vorrichtung kann die Pyrrolopyrrol-Cyanin-Verbindung einer Reinigung unterzogen werden. Die Reinigung kann nach üblichen, dem Fachmann bekannten Verfahren erfolgen, wie Auftrennung an geeigneten stationären Phasen, Sublimation, Extraktion, Destillation, Umkristallisation oder eine Kombination aus wenigstens zwei dieser Maßnahmen. Jede Reinigung kann dabei ein- oder mehrstufig ausgestaltet sein.

Die eingesetzten Pyrrolopyrrol-Cyanine der allgemeinen Formel I eignen sich besonders vorteilhaft für einen Einsatz in der organischen Photovoltaik (OPV). Sie übernehmen üblicherweise die Rolle des Elektronendonors. Aufgrund ihres hohen Absorptionsvermögens ist sogar bei dünner (photo)aktiver Schicht eine hohe Quantenausbeute möglich. Der Begriff "photoaktiv" bezeichnet hierbei die Umwandlung von Lichtenergie in elektrische Energie.

Gemäß einer bevorzugten Ausführungsform ist die Vorrichtung der organischen Elektronik eine organische

Solarzelle mit photoaktiven Donor-Akzeptor-Übergängen.

Die Erfindung stellt somit eine organische Solarzelle mit einem photoaktiven Bereich, der wenigstens ein organisches Donormaterial in Kontakt mit wenigstens einem organischen Akzeptormaterial aufweist, zur Verfügung, wobei das Donormaterial und das Akzeptormaterial einen Donor-Akzeptor-Heteroübergang ausbilden und wobei der photoaktive Bereich wenigstens eine Verbindung der Formel I, wie zuvor definiert, enthält.

Der photoaktive Bereich kann zwei Schichten umfassen, die jeweils eine homogene Zusammensetzung haben und einen "flachen" Donor-Akzeptor-Heteroübergang (flat donor-acceptor heterojunction) ausbilden. Ein photoaktiver Bereich kann auch eine Mischschicht umfassen und einen Donor-Akzeptor Heteroübergang in Form einer Donor-Akzeptor Bulk Heterojunction ausbilden. Organische Solarzellen mit photoaktiven Donor-Akzeptor-Übergängen in Form einer Bulk Heterojunction sind eine bevorzugte Ausführungsform der Erfindung.

Eine weitere Ausführungsform ist die Kombination einer Donor-Akzeptor Bulk Heterojunction, an die eine intrinsische Schicht einer Pyrrolopyrrol-Cyanin-Verbindung der Formel I angrenzt. Die in dieser intrinsischen Schicht generierten Exzitonen können an die Donor-Akzeptor-Mischschicht diffundieren und dort direkt in freie Ladungsträger dissoziiert werden oder es findet ein Energieübertrag auf den Akzeptor oder Donor der Mischschicht statt. Die in und an der Mischschicht generierten Löcher gelangen durch die intrinsische Schicht der Pyrrolopyrrol-Cyanin-Verbindung der Formel I zu dem Kontakt. Hierbei wählt man vorzugsweise ein höchstes besetztes Energieniveau (HOMO) und ein niedrigstes unbesetztes Energieniveau (LUMO) des organischen AkzeptorMaterials (elektronenleitendes Material) und des organischen Donator-Materials (löcherleitendes Material) so, dass eine effiziente Trennung der Exzitonen in Elektronen auf dem Akzeptor-Material und von Löchern auf dem Donator-Material erfolgt. Es ist zudem vorteilhaft, die Energieniveaus des Donormaterials der Donor-Akzeptormischschicht und der intrinsischen Schicht, enthaltend die Pyrrolopyrrol-Verbindung der Formel I in Hinblick auf den Löchertransport aufeinander abzustimmen.

In einer weiteren Ausführungsform der Erfindung ist die organische Solarzelle in Form einer Tandemzelle, wobei eine der Unterzellen (Subzellen) einen photoaktiven Bereich mit wenigstens einer Verbindung der Formel I, wie zuvor definiert, enthält. Vorzugsweise weisen beide Unterzellen einen photoaktiven Donor-Akzeptor-Übergang in Form einer Bulk-Heterojunction auf.

In einer weiteren Ausführungsform der Erfindung ist die organische Solarzelle als Mehrfachzelle ausgeführt, wobei eine der Unterzellen einen photoaktiven Bereich mit wenigstens einer Verbindung der Formel I, wie zuvor definiert, enthält. Vorzugsweise weist wenigstens eine der Unterzellen einen photoaktiven Donor-Akzeptor-Übergang in Form einer Bulk-Heterojunction auf.

In einer weiteren Ausführungsform enthält der photoaktive Bereich eine Verbindung der Formel I, wobei die Verbindung der Formel I Teil einer Dreifachmischschicht ist und der Ladungstransport insbesondere von den beiden anderen Komponenten bereitgestellt wird.

Organische Solarzellen sind im Allgemeinen schichtförmig aufgebaut und umfassen in der Regel zumindest die folgenden Schichten: Anode, photoaktiver Bereich und Kathode. Diese Schichten werden im Allgemeinen auf ein für diesen Zweck übliches Substrat aufgetragen. Die Struktur organischer Solarzellen ist z. B. in US 2005/0098726 und US 2005/0224905 beschrieben.

Geeignete Substrate für organische Solarzellen sind z. B. oxidische Materialien, Polymere und Kombinationen davon. Bevorzugte oxidische Materialien sind ausgewählt unter Glas, Keramik, $SiO_2$, Quarz, etc. Bevorzugte Polymere sind ausgewählt unter Polyolefinen (wie Polyethylen und Polypropylen), Polyestern (wie Polyethylenterephthalat und Polyethylennaphthalat), Fluorpolymeren, Polyamiden, Polyurethanen, Polyalkyl(meth)acrylaten, Polystyrolen, Polyvinylchloriden und Mischungen und Kompositen davon.

Als Elektroden (Kathode, Anode) eignen sich prinzipiell Metalle, Halbleiter, Metalllegierungen, Halbleiterlegierungen und Kombinationen davon. Bevorzugte Metalle sind die der Gruppen 2, 9, 10, 11 oder 13 des Periodensystems (PES), z. B. die Erdalkalimetalle (Gruppe 2) Mg, Ca und Ba, der 10. Gruppe des PES wie Pt, der 11. Gruppe des PES wie Au, Ag und Cu und der 13. Gruppe des PES wie Al und In. Bevorzugt sind Metalllegierungen, z. B. auf Basis Pt, Au, Ag, Cu, etc. und spezielle Mg/Ag-Legierungen, des Weiteren aber auch Alkalimetallfluoride, wie LiF, NaF, KF, RbF und CsF, und Mischungen aus Alkalimetallfluoriden und Alkalimetallen. Bevorzugt wird als Elektrode ein das einfallende Licht im Wesentlichen reflektierendes Material eingesetzt. Dazu zählen beispielsweise Metallfilme aus Al, Ag, Au, In, Mg, Mg/Al, Ca, etc.

Bevorzugte Halbleiter sind z. B. dotiertes Si, dotiertes Ge, Indium-Zinn-Oxid (ITO), fluoriertes Zinnoxid (FTO), Gallium-Indium-Zinn-Oxid (GITO), Zink-Indium-Zinn-Oxid (ZITO), etc.

Bevorzugt wird als Material für die dem Licht zugewandte Elektrode (bei normaler Struktur die Anode, bei inverser Struktur die Kathode) ein gegenüber dem einfallendem Licht zumindest teilweise transparentes Material eingesetzt. Dazu zählen vorzugsweise Elektroden, die als Trägermaterial Glas und/oder ein transparentes Polymer aufweisen. Als Träger geeignete transparente Polymere sind die zuvor genannten, wie Polyethylenterephthalat. Die elektrische Kontaktierung erfolgt in der Regel durch Metallschichten und/oder transparente leitfähige Oxide (TCOs). Dazu zählt vorzugsweise ITO, dotiertes ITO, FTO (fluorine doped tin oxide), AZO (aluminium doped tin oxide), ZnO, $TiO_2$, Ag, Au, Pt. Besonders bevorzugt zur Kontaktierung ist ITO. Zur elektrischen Kontaktierung kann auch ein leitfähiges Polymer,

wie z. B. ein Poly-3,4-alkylendioxythiophen, z. B. Poly-3,4-ethylenoxythiophen (PEDOT) eingesetzt werden.

Die dem Licht zugewandte Elektrode wird so ausgeführt, dass sie ausreichend dünn ist, um nur eine minimale Lichtabsorption zu bewirken, aber dick genug, um einen guten Ladungstransport der extrahierten Ladungsträger zu ermöglichen. Die Dicke der Elektrodenschicht (ohne Trägermaterial) liegt vorzugsweise in einem Bereich von 20 bis 200 nm.

In einer speziellen Ausführung wird als Material für die dem Licht abgewandte Elektrode (bei normaler Struktur die Kathode, bei inverser Struktur die Anode) ein das einfallende Licht zumindest teilweise reflektierendes Material eingesetzt. Dazu zählen Metallfilme, vorzugsweise aus Ag, Au, Al, Ca, Mg, In und Mischungen davon. Bevorzugte Mischungen sind Mg/Al. Die Dicke der Elektrodenschicht liegt vorzugsweise in einem Bereich von 50 bis 300 nm.

In einer weiteren Ausführungsform wird als Material für die dem Licht abgewandte Elektrode (bei normaler Struktur die Kathode, bei inverser Struktur die Anode) ein das einfallende Licht nur gering reflektierendes Material eingesetzt. Dies ermöglicht den Aufbau von transparenten Solarzellen. Die Licht abgewandte Elektrode kann ebenfalls durch Metallfilme, vorzugsweise aus Ag, Au, Al, Ca, Mg, In und Mischungen davon, beschichtet werden. Die Dicke der Elektrodenschicht liegt vorzugsweise in einem Bereich von 5 bis 30 nm. Der NIR-Absorptionsbereich von Pyrrolopyrrol-Cyanin-Verbindung ist für transparente Solarzellen von besonderem Interesse, da das menschliche Auge in diesem Bereich nur wenig bis gar nicht empfindlich ist.

Der photoaktive Bereich enthält wenigstens ein organisches Donormaterial in Kontakt mit wenigstens einem organischen Akzeptormaterial, wobei das Donormaterial und das Akzeptormaterial einen Donor-Akzeptor-Heteroübergang ausbilden und wobei der photoaktive Bereich wenigstens eine Verbindung der Formel I aufweist.

Geeignete Akzeptormaterialien sind vorzugsweise ausgewählt aus der Gruppe

a) Fullerene und Fullerenderivate,
b) polycyclische aromatische Kohlenwasserstoffen und deren Derivaten, insbesondere Naphthalin und dessen Derivate, Rylene, insbesondere Perylen, Terrylen und Quaterrylen, und deren Derivate, Azene, insbesondere Anthrazen, Tetrazen, in besondere Rubren, Pentazen und deren Derivate, Pyren und dessen Derivate,
c) Chinone, Chinondimethane und deren Derivaten,
d) Phthalocyanine, Subphthalocyanine und deren Derivaten,
e) Porphyrine, Tetraazoporphyrine, Tetrabenzoporphyrine und deren Derivaten,
f) Thiophene, oligo-Thiophene, kondensierte/anellierte Thiophene, wie Thienothiophen und Bithienothiophen, und deren Derivate,
g) Thiadiazole und deren Derivate,
h) Carbazole und Triarylamine und deren Derivate,
i) Indanthrone, Violanthrone und Flavanthone und deren Derivate und
j) Fulvalene, Tetrathiafulvalene und Tetraselenafulvalene und deren Derivate.

Vorzugsweise ist das Akzeptormaterial ein oder mehrere Fullerene und/oder Fulleren-Derivate. Geeignete Fullerene sind vorzugsweise ausgewählt unter $C_{60}$, $C_{70}$, $C_{76}$, $C_{80}$, $C_{82}$, $C_{84}$, $C_{86}$, $C_{90}$ und $C_{94}$. Geeignete Fullerenderivate sind vorzugsweise ausgewählt unter Phenyl-$C_{61}$-Butyrsäuremethylester ([60]PCBM), Phenyl-$C_{71}$-Butyrsäuremethylester ([71]PCBM), Phenyl-$C_{84}$-Butyrsäuremethylester ([84]PCBM), Phenyl-$C_{61}$-Butyrsäurebutylester ([60]PCBB), Phenyl-$C_{61}$-Butyrsäureoctyylester ([60]PCBO) und Thienyl-$C_{61}$-Butyrsäuremethylester ([60]ThCBM). Besonders bevorzugt sind $C_{60}$, [60]PCBM und Mischungen davon.

Der Gehalt an der Verbindung der Formel I in dem photoaktiven Bereich beträgt vorzugsweise 10 bis 90 Gew.-%, besonders bevorzugt 25 bis 75 Gew.-%, bezogen auf das Gesamtgewicht des Halbleitermaterials (p- und n-Halbleiter) in dem photoaktiven Bereich. Das Akzeptormaterial liegt in einem Anteil von 90 bis 10 Gew.-%, insbesondere 75 bis 25 Gew.-% vor, bezogen auf das Gesamtgewicht des Halbleitermaterials (p- und n-Halbleiter) in dem photoaktiven Bereich.

Der photoaktive Bereich wird so ausgeführt, dass er ausreichend dick ist, um eine maximale Lichtabsorption zu bewirken, aber dünn genug, um die erzeugten Ladungsträger effizient zu extrahieren. Die Gesamtdicke der den photoaktiven Bereich bildenden Schicht(en) liegt vorzugsweise in einem Bereich von 5 bis 200 nm, besonders bevorzugt 10 bis 80 nm.

Zusätzlich zu dem photoaktiven Bereich kann die organische Solarzelle eine oder mehrere weitere Schichten aufweisen, die üblicherweise das einfallende Licht nicht absorbieren, oder auch Schichten, welche als Ladungstransportschichten dienen und gleichzeitig die Kontaktierung zu einer oder beiden Elektroden der Solarzelle verbessern.

Dazu zählen z. B.

- Schichten mit lochleitenden Eigenschaften (hole transport layer, HTL),
- Schichten mit elektronenleitenden Eigenschaften (ETL, electron transport layer),
- Excitonen (und ggf. Löcher) blockierende Schichten (excition blocking layers, EBL),
- Multiplikatorschichten (multiplication layers).

Geeignete Schichten mit lochleitenden Eigenschaften enthalten vorzugsweise wenigstens ein Material mit einer geringen Ionisierungsenergie bezogen auf Vakuumniveau, d. h. die Schicht mit lochleitenden Eigenschaften weist eine kleinere Ionisierungsenergie und eine kleinere Elektronenaffinität, bezogen auf Vakuumniveau, auf als die Schicht mit elektronenleitenden Eigenschaften. Bei den Materialien kann es sich um organische oder anorganische Materialien handeln. Für den Einsatz in einer Schicht mit lochleitenden Eigenschaften geeignete organische Materialien sind vorzugsweise ausgewählt unter Poly(3,4-ethylendioxythiophen)-poly(styrolsulfonat) (PEDOT-PSS), Ir-DPBIC (Tris-N,N'-Diphenylbenzimidazol-2-yliden-iridium(III)), N,N'-diphenyl-N,N'-bis(3-methylphenyl)-1,1'-diphenyl-4,4'-diamin (alpha-NDP), 2,2',7,7'-Tetrakis(N,N-di-p-methoxyphenylamin)-9,9'-spirobifluoren (spiro-MeOTAD), 9,9-Bis[4-(N,N-bis-biphenyl-4-yl-amino)phenyl]-9H-fluoren (BPAPF), N,N-Diphenyl-N,N-bis (4-(N,N-bis(naphth-1-yl)-amino)-biphenyl-4-yl)-benzidin (DiNPB) etc. und Mischungen davon. Geeignete anorganische Materialien sind $WO_3$, $MoO_3$, usw. Für den Fall, dass die Schicht mit lochleitenden Eigenschaften aus organischem Material aufgebaut ist, kann sie mit einem p-Dotierstoff versetzt werden, dessen LUMO-Energie im gleichen oder niedrigeren Energiebereich liegt wie das HOMO der Schicht mit lochleitenden Eigenschaften. Geeignete Dotiermittel sind z. B. organische Dotiermittel oder anorganische Dotiermittel wie 2,3,5,6-Tetrafluor-7,7,8,8-tetracyanochinodimethan ($F_4$TCNQ),r NDP9 (erhältlich von Novaled AG, Dresden), $WO_3$, $MoO_3$, etc.

Soweit vorhanden, liegt die Dicke der Schichten mit lochleitenden Eigenschaften vorzugsweise in einem Bereich von 5 bis 200 nm, besonders bevorzugt 10 bis 100 nm.

Geeignete Schichten mit elektronenleitenden Eigenschaften enthalten vorzugsweise wenigstens ein Material, dessen LUMO, bezogen auf Vakuumniveau, energetisch höher liegt als das LUMO des Materials mit lochleitenden Eigenschaften. Bei den Materialien kann es sich um organische oder anorganische Materialien handeln. Für den Einsatz in einer Schicht mit elektronenleitenden Eigenschaften geeignete organische Materialien sind vorzugsweise ausgewählt unter Fullerenen und Fullerenderivaten wie zuvor definiert, 2,9-Dimethyl-4,7-diphenyl-1,10-phenanthrolin (BCP), 4,7-Diphenyl-1,10-phenanthrolin (Bphen), 1,3-bis[2-(2,2'-bipyridin-6-yl)-1,3,4-oxadiazo-5-yl]benzol (BPY-OXD), etc. Geeignete Fullerene und Fullerenderivate sind vorzugsweise ausgewählt unter $C_{60}$, $C_{70}$, $C_{84}$, Phenyl-$C_{61}$-Butyrsäuremethylester ([60]PCBM), Phenyl-$C_{71}$-Butyrsäuremethylester ([71]PCBM), Phenyl-$C_{84}$-Butyrsäuremethylester ([84]PCBM), Phenyl-$C_{61}$-Butyrsäurebutylester ([60]PCBB), Phenyl-$C_{61}$-Butyrsäureoctyylester ([60]PCBO), Thienyl-$C_{61}$-Butyrsäuremethylester ([60]ThCBM) und Mischungen davon. Besonders bevorzugt sind $C_{60}$, [60]PCBM und Mischungen davon. Ganz besonders bevorzugt enthält die Schicht mit elektronenleitenden Eigenschaften $C_{60}$. Die organischen Materialien können gewünschtenfalls mit einem n-Dotiermittel dotiert werden, welches ein HOMO aufweist, das im gleichen Bereich oder niedriger liegt als das LUMO des elektronenleitenden Materials. Geeignete Dotiermittel sind z. B. $Cs_2CO_3$, Pyronin B (PyB), Rhodamine B, Cobaltocene, etc. Für den Einsatz in einer Schicht mit elektronenleitenden Eigenschaften geeignete anorganische Materialien sind vorzugsweise ausgewählt unter ZnO, etc. Besonders bevorzugt enthält die Schicht mit elektronenleitenden Eigenschaften $C_{60}$.

Soweit vorhanden, liegt die Dicke der Schichten mit elektronenleitenden Eigenschaften vorzugsweise in einem Bereich von 5 bis 200 nm, besonders bevorzugt 10 bis 100 nm.

Geeignete Excitonen und Löcher blockierende Schichten sind z. B. in US 6,451,415 beschrieben. Geeignete Materialien für Excitonenblockerschichten sind z. B. 2,9-Dimethyl-4,7-diphenyl-1,10-phenanthrolin (BCP), 4,7-Diphenyl-1,10-phenanthrolin (Bphen), 1,3-bis[2-(2,2'-bipyridin-6-yl)-1,3,4-oxadiazo-5-yl]benzol (BPY-OXD), Polyethylendioxythiophen (PEDOT), etc. Bevorzugt wird ein Material eingesetzt, dass gleichzeitig gut für einen Elektronentransport geeignet ist. Bevorzugt sind BCP, Bphen und BPY-OXD.

Soweit vorhanden, liegt die Dicke der Schichten mit excitonenblockierenden Eigenschaften vorzugsweise in einem Bereich von 1 bis 50 nm, besonders bevorzugt 2 bis 20 nm.

Die erfindungsgemäßen Solarzellen umfassen wenigstens einen photoaktiven Donor-Akzeptor-Heteroübergang (donor-acceptor heterojunction). Durch optische Anregung eines organischen Materials werden Excitonen erzeugt. Damit ein Photostrom auftritt, muss das Elektron-Loch-Paar getrennt werden, üblicherweise an einer Donor-Akzeptor-Grenzfläche zwischen zwei ungleichen Kontaktmaterialien. An solch einer Grenzfläche bildet das Donormaterial einen Heteroübergang mit einem Akzeptormaterial. Werden die Ladungen nicht separiert, so können sie in einem auch als "Quenchen" bezeichneten Prozess rekombinieren, entweder strahlend durch die Emission von Licht einer niedrigeren Energie als das eingestrahlte oder nichtstrahlend durch Erzeugung von Wärme. Beide Prozesse sind unerwünscht.

In einer ersten Ausführung ist der Heteroübergang flach ausgeführt. (siehe: Two layer organic photovoltaic cell, C. W. Tang, Appl. Phys. Lett., 48 (2), 183-185 (1986) oder N. Karl, A. Bauer, J. Holzäpfel, J. Marktanner, M. Möbus, F. Stölzle, Mol. Cryst. Liq. Cryst., 252, 243-258 (1994).).

In einer zweiten bevorzugten Ausführung ist der Heteroübergang als bulk (mixed) heterojunction ausgeführt, auch als interpenetrierendes Donor-Akzeptor-Netzwerk bezeichnet. Organische photovoltaische Zellen mit einem bulk heterojunction werden z. B. von C. J. Brabec, N. S. Sariciftci, J. C. Hummelen in Adv. Funct. Mater., 11 (1), 15 (2001) oder by J. Xue, B. P. Rand, S. Uchida und S. R. Forrest in J. Appl. Phys. 98, 124903 (2005) beschrieben.

Die Prozessierung der photoaktiven Schicht kann aus Lösung erfolgen. Hierbei kann die Verbindung der Formel I und wenigstens ein Elektronenakzeptormaterial bereits gemeinsam gelöst, aber auch separat als Lösung der Verbindung der Formel I und Lösung des Elektronenakzeptormaterials vorliegen, wobei die Vermischung der entsprechenden Lösungen in letzterem Fall kurz vor der Aufbringung auf die darunterliegende Schicht erfolgt. Als Lösungsmittel eignen sich alle Lösungsmittel, die rückstandsfrei verdampfen und eine ausreichende Löslichkeit für die Verbindung der Formel I und das Elektronenakzeptormaterial aufweisen. Geeignete Lösungsmittel sind aromatische Verbindungen wie Benzol, Toluol, Xylole, Chlorbenzol, Dichlorbenzole, stickstoffhaltige Heterocyclen, cyclische Ether, wie etwa Tetrahydrofuran, Ketone wie Aceton, halogenierte Aliphaten wie Trichlormethan, lineare oder cyclische Kohlenwasserstoffe wie Hexan oder Cyclohexan oder Alkohole, wie etwa Methanol, Ethanol, Propanol, Isopropanol oder Butanol, und Mischungen davon.

Geeignete Methoden zur Aufbringung der photoaktiven Schichten aus flüssiger Phase sind dem Fachmann bekannt. Vorteilhaft zeigt sich hier insbesondere die Prozessierung mittels Spin-Coating, da die Dicke der photoaktiven Schicht in einfacher Weise durch die Menge und/oder Konzentration der verwendeten Lösung sowie die Rotationsgeschwindigkeit und/oder Rotationsdauer gesteuert werden kann. Die Prozessierung der Lösung erfolgt in der Regel bei Raumtemperatur.

Vorzugsweise erfolgt die Abscheidung der photoaktiven Schicht aber aus der Gasphase, insbesondere durch Vakuumsublimation. Üblicherweise werden für die Abscheidung Temperaturen zwischen 100 bis 600°C angewendet, je nach Stabilität der Verbindung der Formel I und des Akzeptormaterials. Die Abscheidung erfolgt vorzugsweise bei einem Druck im Bereich von etwa $10^{-2}$ bis $10^{-8}$ mbar, z. B. $10^{-5}$ bis $10^{-8}$ mbar.

In einer bevorzugten Ausführungsform erfolgt die Herstellung der photoaktiven Donor-Akzeptor-Übergänge in Form einer Bulk-Heterojunction durch ein Gasphasenabscheidungsverfahren (Physical Vapor Deposition, PVD). Geeignete Verfahren sind z. B. in der US 2005/0227406 beschrieben, worauf hier Bezug genommen wird. Dazu kann die Verbindung der Formel I und wenigstens ein Elektronenakzeptormaterial einer Gasphasenabscheidung im Sinne einer Cosublimation unterzogen werden. PVD-Verfahren werden unter Hochvakuumbedingungen durchgeführt und umfassen die folgenden Schritte: Verdampfen, Transport, Abscheidung.

Die Abscheidung erfolgt vorzugsweise bei einem Druck im Bereich von etwa $10^{-2}$ bis $10^{-8}$ mbar, z. B. $10^{-5}$ bis $10^{-8}$ mbar.

Die Abscheidungsrate liegt vorzugsweise in einem Bereich von etwa 0,01 bis 10 nm/s.

Die Temperatur des Substrats bei der Abscheidung liegt vorzugsweise in einem Bereich von etwa -100 bis 250 °C, besonders bevorzugt -50 bis 150 °C. Die Abscheidung kann unter einer Inertgasatmosphäre, z. B. unter Stickstoff, Argon oder Helium erfolgen.

Die Herstellung der übrigen, die Solarzelle bildenden Schichten kann nach üblichen, dem Fachmann bekannten Verfahren erfolgen. Dazu zählt das Aufdampfen im Vakuum oder in Inertgasatmosphäre, Laserablation oder Lösungs- oder Dispersionsprozessierverfahren wie Spin-Coating, Rakeln, Gießverfahren, Aufsprühen, Tauchbeschichtung oder Drucken (z. B. Inkjet, Flexo, Offset, Gravur; Tiefdruck, Nanoimprint). Bevorzugt erfolgt die Herstellung der gesamten Solarzelle durch ein Gasphasenabscheidungsverfahren.

Die photoaktive Schicht (Mischschicht) kann direkt nach ihrer Herstellung oder nach Herstellung weiterer die Solarzelle bildenden Schichten einer thermischen Behandlung unterzogen werden. Durch ein solches Tempern kann vielfach die Morphologie der photoaktiven Schicht noch verbessert werden. Die Temperatur liegt vorzugsweise in einem Bereich von etwa 60 °C bis 200 °C. Die Behandlungsdauer liegt vorzugsweise in einem Bereich von 1 Minute bis 3 Stunden. In Ergänzung oder alternativ zu einer thermischen Behandlung kann die photoaktive Schicht direkt nach ihrer Herstellung oder nach Herstellung weiterer die Solarzelle bildenden Schichten einer Behandlung mit einem lösungsmittelhaltigen Gas unterzogen werden. In einer geeigneten Ausführung werden gesättigte Lösungsmitteldämpfe in Luft bei Umgebungstemperatur eingesetzt. Geeignete Lösungsmittel sind Toluol, Xylol, Chloroform, N-Methylpyrrolidon, Dimethylformamid, Ethylacetat, Chlorbenzol, Dichlormethan und Mischungen davon. Die Behandlungsdauer liegt vorzugsweise in einem Bereich von 1 Minute bis 3 Stunden.

Die erfindungsgemäßen Solarzellen können als einzelne Zelle mit normaler Struktur vorliegen. In einer speziellen Ausführungsform weist eine solche Zelle den folgenden Schichtaufbau auf:

- ein zumindest teilweise lichtdurchlässiges Substrat,
- eine erste Elektrode (Frontelektrode, Anode)
- eine lochleitende Schicht,
- eine photoaktive Schicht,
- elektronenleitende Schicht,
- eine excitonenblockierende/elektronenleitende Schicht,
- eine zweite Elektrode (Rückelektrode, Kathode).

Die erfindungsgemäßen Solarzellen können auch als einzelne Zelle mit inverser Struktur vorliegen. In einer speziellen Ausführungsform weist eine solche Zelle den folgenden Schichtaufbau auf:

- ein zumindest teilweise lichtdurchlässiges Substrat,
- eine erste Elektrode (Frontelektrode, Kathode)
- eine excitonenblockierende/elektronenleitende Schicht,
- elektronenleitende Schicht,
- eine photoaktive Schicht,
- eine lochleitende Schicht,
- eine zweite Elektrode (Rückelektrode, Anode).

Die Verbindungen der Formel I können als photoaktives Material in Zellen mit MiM, pin, pn, Mip oder Min Struktur eingesetzt werden (M = metal, p = p-doped organic or inorganic semiconductor, n = n-doped organic or inorganic semiconductor, i = intrinsically conductive system of organic layers; siehe z. B. J. Drechsel et al., Org. Electron., 5 (4), 175 (2004) oder Maennig et al., Appl. Phys. A 79, 1-14 (2004)).

Die Verbindungen der Formel I können auch als photoaktives Material in Tandemzellen eingesetzt werden. Geeignete Tandemzellen werden z. B. von P. Peumans, A. Yakimov, S. R. Forrest in J. Appl. Phys, 93 (7), 3693-3723 (2003) beschrieben (siehe auch US 4,461,922, US 6,198,091 und US 6,198,092) und werden im Folgenden detailliert beschrieben. Der Einsatz von Pyrrolopyrrol-Cyaninen der allgemeinen Formel I in Tandemzellen ist eine bevorzugte Ausführungsform der Erfindung.

Eine Tandemzelle besteht aus zwei oder mehr als zwei (z. B. 3, 4, 5, etc.) Unterzellen. Eine Tandemzelle mit mehr als zwei Unterzellen wird auch als Mehrfachsolarzelle beschrieben. Dabei können eine einzelne Unterzelle, ein Teil der Unterzellen oder alle Unterzellen photoaktive Donor-Akzeptor-Heteroübergänge aufweisen. Jeder Donor-Akzeptor-Heteroübergang kann in Form einer flat heterojunction, in Form einer Bulk-Heterojunction in Form einer oben beschriebenen Kombination aus Bulk-Heterojunction und angrenzender intrinsischen Absorberschicht, in Form einer Kombination aus mehr als einer Donor-Akzeptor Mischschicht oder in Form einer Dreifachmischschicht orliegen. Erfindungsgemäß weist der photoaktive Bereich wenigstens einer Unterzelle eine Pyrrolopyrrol-Cyanin-Verbindung der allgemeinen Formel I auf. Bevorzugt umfasst der photoaktive Bereich wenigstens einer Unterzelle eine Pyrrolopyrrol-Cyanin-Verbindung der allgemeinen Formel I und wenigstens ein Fulleren oder Fullerenderivat. Besonders bevorzugt besteht die in dem photoaktiven Bereich wenigstens einer Unterzelle eingesetzte Halbleitermischung aus einer Pyrrolopyrrol-Cyanin-Verbindung der allgemeinen Formel I und $C_{60}$-Fulleren. Bevorzugt enthält der photoaktive Bereich einer weiteren Unterzelle Kombinationen anderer Materialien, z. B. eine Verbindung mit einem Absorptionsmaximum kleiner 650 nm, z. B. im UV/VIS-Bereich, und $C_{60}$-Fulleren. Das Hintereinanderschalten von Unterzellen mit unterschiedlicher Bandlücke ermöglicht eine effektive Ausnutzung eines breiten Spektralbereichs. Geeignete Kombinationen anderer Materialien sind Dibenzotetraphenylperiflanthen/$C_{60}$-Fulleren, Oligothiophen(beispielweise DCV5T)/$C_{60}$-Fulleren, Zinkphthalocyanin/$C_{60}$-Fulleren, oder eine der Unterzellen ist eine farbstoff-sensibilisierte Solarzelle oder eine Polymerzelle, wie beispielsweise in der Kombination Poly(3-hexyl-thiophen)/PCBM. Vorzugsweise wird die Kombination der Materialien/Unterzellen so gewählt wird, dass die Lichtabsorptionen der Unterzellen nicht zu stark überlappen, sondern in Summe das Spektrum des Sonnenlichts abdecken, was zu einer Erhöhung der Stromausbeute führt. Um die Leistung der Tandem-Solarzelle zu optimieren, passt man üblicherweise die von den Subzellen bereitgestellten Ströme unter Betriebsbedingungen aufeinander an. Um das Spektrum des einfallenden Lichts besser auszunutzen, enthält vorzugsweise der photoaktive Bereich beider Subzelle einen Donor-Akzeptor-Heteroübergang in Form einer Bulk-Heterojunction und der photoaktive Bereich einer Unterzelle enthält wenigstens eine Verbindung der Formel I.

Die Verbindungen der Formel I können auch als photoaktives Material in Tandemzellen eingesetzt werden, die aus zwei oder mehr als zwei gestapelten MiM, pin, Mip oder Min Strukturen aufgebaut sind (siehe DE 103 13 232.5 und J. Drechsel et al., Thin Solid Films, 451452, 515-517 (2004)).

Die Schichtdicke der M, n, i und p Schichten liegt üblicherweise in einem Bereich von 10 bis 1000 nm, besonders bevorzugt von 10 bis 400 nm. Die Herstellung der die Solarzelle bildenden Schichten kann nach üblichen, dem Fachmann bekannten Verfahren erfolgen. Dazu zählt das Aufdampfen im Vakuum oder in Inertgasatmosphäre, Laserablation oder Lösungs- oder Dispersionsprozessierverfahren wie Spin-Coating, Rakeln, Gießverfahren, Aufsprühen, Tauchbeschichtung oder Drucken (z. B. Inkjet, Flexo, Offset, Gravur; Tiefdruck, Nanoimprint). In einer speziellen Ausführung erfolgt die Herstellung der gesamten Solarzelle durch ein Gasphasenabscheidungsverfahren.

Die erfindungsgemäßen Solarzellen weisen eine sehr hohe Leerlaufspannung und einen hohen Füllfaktor auf. Zusätzlich zeigen die erfindungsgemäßen Solarzellen eine erstaunlich hohe Lebensdauer, welche sich aus der hohen Stabilität der Verbindungen der allgemeinen Formel I ergibt.

Photodetektoren besitzen im Wesentlichen einen zu organischen Solarzellen analogen Aufbau, sie werden jedoch mit einer Vorspannung betrieben, welche bei Einwirkung von Strahlungsenergie einen entsprechenden Stromfluss als Messantwort generiert.

Ein weiterer Gegenstand der Erfindung ist eine organische Leuchtdiode (OLED), enthaltend eine Anode (An), eine Kathode (Ka) und eine zwischen der Anode und Kathode angeordnete Licht emittierende Schicht (E) sowie gegebenenfalls mindestens eine weitere Schicht ausgewählt aus der Gruppe bestehend aus einer Blockschicht für Elektronen/Excitonen, mindestens einer Blockschicht für Löcher/Excitonen, mindestens einer Loch-Injektionsschicht, mindestens einer Lochleiterschicht, mindestens einer Elektroneninjektionsschicht und mindestens einer Elektronenleiterschicht, wobei mindestens eine Verbindung der allgemeinen Formel I, wie zuvor definiert, in der Licht emittierenden Schicht und/oder in der mindestens einen weiteren Schicht enthalten ist.

Die erfindungsgemäße OLED kann z. B. - in einer bevorzugten Ausführungsform - aus den folgenden Schichten aufgebaut sein:

1. Anode
2. Lochleiterschicht
3. lichtemittierende Schicht
4. Blockschicht für Löcher/Excitonen
5. Elektronenleiterschicht
6. Kathode

Es sind auch von dem vorstehend genannten Aufbau verschiedene Schichtenfolgen möglich, die dem Fachmann bekannt sind. Beispielsweise ist es möglich, dass die OLED nicht alle der genannten Schichten aufweist, z. B. ist eine OLED mit den Schichten (1) (Anode), (3) (lichtemittierende Schicht) und (6) (Kathode) ebenfalls geeignet, wobei die Funktionen der Schichten (2) (Lochleiterschicht) und (4) (Blockschicht für Löcher/Excitonen) und (5) (Elektronenleiterschicht) durch die angrenzenden Schichten übernommen werden. OLEDs, die die Schichten (1), (2), (3) und (6) bzw. die Schichten (1), (3), (4), (5) und (6) aufweisen, sind ebenfalls geeignet. Des Weiteren können die OLEDs zwischen der Anode (1) und der Lochleiterschicht (2) eine Blockschicht für Elektronen/Excitonen aufweisen.

Es ist des Weiteren möglich, dass mehrere der vorstehend genannten Funktionen (Elektronen-/Excitonenblocker, Loch-/Excitonenblocker, Loch-Injektion, Lochleitung, Elektroneninjektion, Elektronenleitung) in einer Schicht vereint sind und z. B. von einem einzigen in dieser Schicht vorliegenden Material übernommen werden. Beispielsweise kann ein in der Lochleiterschicht eingesetztes Material in einer Ausführungsform gleichzeitig Excitonen und/oder Elektronen blocken.

Des Weiteren können die einzelnen der vorstehend genannten Schichten der OLED wiederum aus zwei oder mehreren Schichten aufgebaut sein. Beispielsweise kann die Lochleiterschicht aus einer Schicht aufgebaut sein, in die aus der Elektrode Löcher injiziert werden, und einer Schicht, die die Löcher von der Loch-injizierenden Schicht weg in die lichtemittierende Schicht transportiert. Die Elektronenleitungsschicht kann ebenfalls aus mehreren Schichten bestehen, z. B. einer Schicht, worin Elektronen durch die Elektrode injiziert werden, und einer Schicht, die aus der ElektronenInjektionsschicht Elektronen erhält und in die lichtemittierende Schicht transportiert.

In einer speziellen Ausführungsform enthält die erfindungsgemäße OLED eine Lochleiterschicht, die wenigstens eine Verbindung der Formel I enthält. Diese Lochleiterschicht kann als einzige Lochleiterschicht oder in Kombination mit wenigstens einer weiteren Lochleiterschicht eingesetzt werden.

Die genannten Schichten werden jeweils nach Faktoren wie Energieniveau, Temperaturresistenz und Ladungsträgerbeweglichkeit, sowie Energiedifferenz der genannten Schichten mit den organischen Schichten oder den Metallelektroden ausgewählt. Der Fachmann ist in der Lage, den Aufbau der OLEDs so zu wählen, dass er optimal an die als Emittersubstanzen verwendeten Verbindungen angepasst ist.

Um besonders effiziente OLEDs zu erhalten, sollte z. B. das HOMO (höchstes besetztes Molekülorbital) der Lochleiterschicht mit der Arbeitsfunktion der Anode angeglichen sein, und das LUMO (niedrigstes unbesetztes Molekülorbital) der Elektronenleiterschicht sollte mit der Arbeitsfunktion der Kathode angeglichen sei, soweit die vorstehend genannten Schichten in den erfindungsgemäßen OLEDs vorliegen.

Gegenstand der Erfindung sind weiterhin organische Feldeffekttransistoren, umfassend ein Substrat mit wenigstens einer Gate-Struktur, einer Source-Elektrode und einer Drain-Elektrode und wenigstens einer Verbindung der Formel I, wie zuvor definiert, als Dotierstoff. Gegenstand der Erfindung sind weiterhin Substrate mit einer Vielzahl von organischen Feldeffekttransistoren, wobei zumindest ein Teil der Feldeffekttransistoren wenigstens eine Verbindung der Formel I, wie zuvor definiert, als Dotierstoff enthält. Gegenstand der Erfindung sind auch Halbleiterbausteine, die wenigstes ein solches Substrat umfassen.

Eine spezielle Ausführungsform ist ein Substrat mit einem Muster (Topographie) von organischen Feldeffekttransistoren, wobei jeder Transistor

- einen auf dem Substrat befindlichen organischen Halbleiter;
- eine Gate-Struktur zur Steuerung der Leitfähigkeit des leitenden Kanals; und
- leitfähige Source- und Drain-Elektroden an den beiden Enden des Kanals

enthält, wobei der organische Halbleiter wenigstens eine Verbindung der Formel I umfasst. Des Weiteren umfasst der organische Feldeffekttransistor in der Regel ein Dielektrikum.

Eine weitere spezielle Ausführungsform ist ein Substrat mit einem Muster von organischen Feldeffekttransistoren, wobei jeder Transistor einen integrierten Schaltkreis bildet oder Teil eines integrierten Schaltkreises ist und wobei zumindest ein Teil der Transistoren wenigstens eine Verbindung der Formel I umfasst.

Als Substrate eignen sich prinzipiell die dafür bekannten Materialien. Geeignete Substrate umfassen z. B. Metalle (vorzugsweise Metalle der Gruppen 8, 9, 10 oder 11 des Periodensystems, wie Au, Ag, Cu), oxidische Materialien (wie Glas, Quarz, Keramiken, $SiO_2$), Halbleiter (z. B. gedoptes Si, gedoptes Ge), Metalllegierungen (z. B. auf Basis von Au, Ag, Cu, etc.), Halbleiterlegierungen, Polymere (z. B. Polyvinylchlorid, Polyolefine, wie Polyethylen und Polypropylen, Polyester, Fluoropolymere, Polyamide, Polyimide, Polyurethane, Polyalkyl(meth)acrylate, Polystyrol und Mischungen und Komposite davon), anorganische Feststoffe (z. B. Ammoniumchlorid), Papier und Kombinationen davon. Die Substrate können flexibel oder unflexibel solid, mit gekrümmter oder planarer Geometrie sein, abhängig von der gewünschten Anwendung.

Ein typisches Substrat für Halbleiterbausteine umfasst eine Matrix (z. B. eine Quarz- oder Polymermatrix) und, optional, eine dielektrische Deckschicht.

Geeignete Dielektrika sind $SiO_2$, Polystyrol, Poly-$\alpha$-methylstyrol, Polyolefine (wie Polypropylen, Polyethylen, Polyisobuten), Polyvinylcarbazol, fluorierte Polymere (z. B. Cytop, CYMM) Cyanopullane, Polyvinylphenol, Poly-p-xylol, Polyvinylchlorid oder thermisch oder durch Luftfeuchtigkeit vernetzbare Polymere. Spezielle Dielektrika sind "self assembled nanodielectrics", d. h. Polymere, welche aus SiCl-Funktionalitäten enthaltenden Monomeren wie z. B. $Cl_3SiOSiCl_3$, $Cl_3Si-(CH_2)_6-SiCl_3$, $Cl_3Si-(CH_2)_{12}-SiCl_3$ erhalten und/oder welche durch Luftfeuchtigkeit oder durch Zugabe von Wasser in Verdünnung mit Lösungsmitteln vernetzt werden (siehe z. B. Faccietti Adv. Mat. 2005, 17, 1705-1725). Anstelle von Wasser können auch hydroxylgruppenhaltige Polymere wie Polyvinylphenol oder Polyvinylalkohol oder Copolymere aus Vinylphenol und Styrol als Vernetzungskomponenten dienen. Es kann auch wenigstens ein weiteres Polymer während des Vernetzungsvorgangs zugegen sein, wie z. B. Polystyrol, welches dann mitvernetzt wird (siehe Facietti, US-Patentanmeldung 2006/0202195).

Das Substrat kann zusätzlich Elektroden aufweisen, wie Gate-, Drain- und Source-Elektroden von OFETs, die normalerweise auf dem Substrat lokalisiert sind (z. B. abgeschieden auf oder eingebettet in eine nichtleitende Schicht auf dem Dielektrikum). Das Substrat kann zusätzlich leitfähige Gate-Elektroden der OFETs enthalten, die üblicherweise unterhalb der dielektrischen Deckschicht (d. h. dem Gate-Dielektrikum) angeordnet sind.

Nach einer speziellen Ausführung befindet sich eine Isolatorschicht (gate insulating layer) auf wenigstes einem Teil der Substratoberfläche. Die Isolatorschicht umfasst wenigstens einen Isolator, der vorzugsweise ausgewählt ist unter anorganischen Isolatoren, wie $SiO_2$, SiN, etc., ferroelektrischen Isolatoren, wie $Al_2O_3$, $Ta_2O_5$, $La_2O_5$, $TiO_2$, $Y_2O_3$, etc., organischen Isolatoren, wie Polyimiden, Benzocyclobuten (BCB), Polyvinylalkoholen, Polyacrylaten, etc. und Kombinationen davon.

Geeignete Materialien für Source- und Drain-Elektroden sind prinzipiell elektrisch leitfähige Materialien. Dazu zählen Metalle, vorzugsweise Metalle der Gruppen 8, 9, 10 oder 11 des Periodensystems, wie Pd, Au, Ag, Cu, Al, Ni, Cr, etc. Geeignet sind weiterhin leitfähige Polymere, wie PEDOT (=Poly(3,4-ethylendioxythiophen); PSS (= Poly(styrolsulfonat), Polyanilin, oberflächenmodifiziertes Gold, etc. Bevorzugte elektrisch leitfähige Materialien haben einen spezifischen Widerstand von weniger als $10^{-3}$, vorzugsweise weniger als $10^{-4}$, insbesondere weniger als $10^{-6}$ oder $10^{-7}$ Ohm x Meter.

Nach einer speziellen Ausführung befinden sich Drain- und Source-Elektroden zumindest teilweise auf dem organischen Halbleitermaterial. Selbstverständlich kann das Substrat weitere Komponenten umfassen, wie sie üblicherweise in Halbleitermaterialien oder ICs eingesetzt werden, wie Isolatoren, Widerstände, Kondensatoren, Leiterbahnen, etc.

Die Elektroden können nach üblichen Verfahren, wie Verdampfen, lithographische Verfahren oder einen anderen Strukturierungsprozess aufgebracht werden.

Die Halbleitermaterialien können auch mit geeigneten Hilfsmitteln (Polymere, Tenside) in disperser Phase durch Verdrucken verarbeitet werden.

In einer bevorzugten Ausführungsform erfolgt die Abscheidung wenigstens einer Verbindung der allgemeinen Formel I (und gegebenenfalls wenigstens einem organischen Halbleitermaterial) durch ein Gasphasenabscheidungsverfahren (Physical Vapor Deposition PVD), wie zuvor beschrieben.

Die erhaltenen Halbleiterschichten weisen im Allgemeinen eine Dicke auf, die für einen ohmschen Kontakt zwischen Source- und Drain-Elektrode ausreicht. Die Abscheidung kann unter einer Inertatmosphäre, z. B. unter Stickstoff, Argon oder Helium, erfolgen.

Die Abscheidung erfolgt üblicherweise bei Umgebungsdruck oder unter reduziertem Druck. Ein geeigneter Druckbereich beträgt etwa $10^{-7}$ bis 1,5 bar.

Vorzugsweise wird die wenigstens eine Verbindung der Formel I enthaltende Schicht auf dem Substrat in einer Dicke von 10 bis 1000 nm, besonders bevorzugt 15 bis 250 nm, abgeschieden.

Die Verbindungen der allgemeinen Formel I können auch vorteilhaft aus Lösung prozessiert werden. Das Auftragen wenigstens einer Verbindung der allgemeinen Formel I auf ein Substrat (und gegebenenfalls wenigstens

eines Halbleitermaterials) erfolgt dann z. B. durch eine Rotationsbeschichtung (spin coating). Die Verbindungen der Formel I eignen sich auch zur Herstellung von Halbleiterelementen, speziell OFETs, durch ein Druckverfahren. Es können dafür übliche Druckprozesse (Inkjet, Flexo, Offset, gravure; Tiefdruck, Nanoprint) verwendet werden. Bevorzugte Lösungsmittel für den Einsatz der Verbindungen der Formel I in einem Druckverfahren sind aromatische Lösungsmittel wie Toluol, Xylol, etc. Man kann zu diesen "Halbleitertinten" verdickend wirkende Substanzen, wie Polymere zusetzen, z. B. Polystyrol, etc. Dabei verwendet man als Dielektrikum die zuvor genannten Verbindungen.

In einer speziellen Ausführungsform handelt es sich bei dem erfindungsgemäßen Feldeffekttransistor um einen Dünnschichttransistor (thin film transistor, TFT). Gemäß einem üblichen Aufbau verfügt ein Dünnschichttransistor über eine auf dem Substrat befindliche Gate-Elektrode, eine auf dieser und dem Substrat befindliche Gate-Isolierschicht, eine auf der Gate-Isolierschicht befindliche Halbleiterschicht, eine ohmsche Kontaktschicht auf der Halbleiterschicht sowie über eine Source-Elektrode und eine Drain-Elektrode auf der ohmschen Kontaktschicht.

In einer geeigneten Ausführungsform wird die Oberfläche des Substrats vor der Abscheidung wenigstens einer Verbindung der allgemeinen Formel I (und gegebenenfalls wenigstens eines organischen Halbleitermaterials) einer Modifizierung unterzogen. Diese Modifizierung dient der Bildung von Dotierstoffe und/oder Halbleitermaterialien bindenden Bereichen und/oder von Bereichen, auf denen keine Dotierstoffe und/oder Halbleitermaterialien abgeschieden werden können. Bevorzugt wird die Oberfläche des Substrats mit wenigstens einer Verbindung (C1) modifiziert, die geeignet ist, an die Oberfläche des Substrats sowie die Verbindungen der Formel I zu binden. In einer geeigneten Ausführungsform wird ein Teil der Oberfläche oder die komplette Oberfläche des Substrats mit wenigstens einer Verbindung (C1) beschichtet, um eine verbesserte Abscheidung wenigstens einer Verbindung der allgemeinen Formel I (und gegebenenfalls weiterer halbleitender Verbindungen) zu ermöglichen. Eine weitere Ausführungsform umfasst die Abscheidung eines Musters von Verbindungen der allgemeinen Formel (C1) auf dem Substrat nach einem entsprechenden Herstellungsverfahren. Dazu zählen die dafür bekannten Maskenprozesse sowie so genannte "Patterning"-Verfahren, wie sie z. B. in der US 2007/0190783 beschrieben sind, worauf hier in vollem Umfang Bezug genommen wird.

Geeignete Verbindungen der Formel (C1) sind zu einer bindenden Wechselwirkung sowohl mit dem Substrat als auch mit wenigstens einem Dotierstoff der allgemeinen Formel I und/oder wenigstens einer Halbleiterverbindung befähigt. Der Begriff "bindende Wechselwirkung" umfasst die Bildung einer chemischen Bindung (kovalenten Bindung), ionischen Bindung, koordinativen Wechselwirkung, Van der Waals-Wechselwirkungen, z. B. Dipol-Dipol-Wechselwirkungen) etc. und Kombinationen davon. Geeignete Verbindungen der allgemeinen Formel (C1) sind:

- Silane, Phosphonsäuren, Carbonsäuren, Hydroxamsäuren, wie Alkyltrichlorsilane, z. B. n-(Octadecyl)trichlorsilan; Verbindungen mit Trialkoxysilan-Gruppen, z. B. Alkyltrialkoxysilane, wie n-Octadecyltrimethoxysilan, n-Octadecyltriethoxysilan, n-Octadecyltri-(n-propyl)oxysilan, n-Octadecyltri-(isopropyl)oxysilan; Trialkoxyaminoalkylsilane, wie Triethoxyaminopropylsilan und N[(3-triethoxysilyl)-propyl]-ethylendiamin; Trialkoxyalkyl-3-glycidylethersilane, wie Triethoxypropyl-3-glycidylethersilan; Trialkoxyallylsilane wie Allyltrimethoxysilan; Trialkoxy-(isocyanatoalkyl)silane; Trialkoxysilyl(meth)acryloxyalkane und Trialkoxysilyl-(meth)acrylamidoalkane, wie 1-Triethoxysilyl-3-acryloxypropan.

- Amine, Phosphine und Schwefel enthaltende Verbindungen, speziell Thiole.

Bevorzugt ist die Verbindung (C1) ausgewählt unter Alkyltrialkoxysilanen, speziell n-Octadecyltrimethoxysilan, n-Octadecyltriethoxysilan; Hexaalkyldisilazanen, und speziell Hexamethyldisilazan (HMDS); $C_8$-$C_{30}$-Alkylthiolen, speziell Hexadecanthiol; Mercaptocarbonsäuren und Mercaptosulfonsäuren speziell Mercaptoessigsäure, 3-Mercaptopropionsäure, Mercaptobernsteinsäure, 3-Mercapto-1-propansulfonsäure und den Alkalimetall- und Ammoniumsalzen davon.

Es sind auch verschiedene Halbleiterarchitekturen mit den erfindungsgemäßen Halbleitern denkbar wie z. B. Top Contact, Top Gate, Bottom Contact, Bottom Gate, oder aber ein vertikaler Aufbau wie z. B. ein VOFET (Vertical organic field effect transistor) wie z. B. in US 2004/0046182 beschrieben.

Die Schichtdicken betragen bei Halbleitern z. B. 10 nm bis 5 $\mu$m, beim Dielektrikum 50 nm bis 10 $\mu$m, die Elektroden können z. B. 20 nm bis 1 $\mu$m dick sein. Die OFETs können auch zu anderen Bauteilen wie Ringoszillatoren oder Inverter kombiniert werden.

Ein weiterer Aspekt der Erfindung ist die Bereitstellung elektronischer Bauteile, die mehrere Halbleiterkomponenten umfassen, wobei es sich um n- und/oder p-Halbleiter handeln kann. Beispiele solcher Bauteile sind Feldeffekttransistoren (FETs), bipolare Flächentransistoren (bipolar junction transistors, BJTs), Tunneldioden, Wechselrichter, lichtemittierende Bauteile, biologische und chemische Detektoren oder Sensoren, temperaturabhängige Detektoren, Photodetektoren wie Polarisations-sensitive Photodetektoren, Gatter, AND-, NAND-, NOT-, OR-, TOR-, und NOR-Gatter, Register, Schalter, Zeitbausteine, statische oder dynamische Speicher und andere dynamische oder sequentielle logische oder andere digitale Bauteile einschließlich programmierbarer Schaltungen.

Ein spezielles Halbleiterelement ist ein Inverter. In der digitalen Logic ist der Inverter ein Gatter, das ein Eingangssignal invertiert. Der Inverter wird auch als NOT-Gate bezeichnet. Reale Inverterschaltungen weisen einen Ausgangsstrom auf, der das Gegenteil zum Eingangsstrom darstellt. Übliche Werte sind z. B. (0, +5V) für TTL-Schaltungen. Die Leistungsfähigkeit eines digitalen Inverters gibt die Spannungstransferkurve (Voltage Transfer Curve VTC) wieder, d. h. der Auftrag von Inputstrom gegen Outputstrom. Idealerweise handelt es sich um eine Stufenfunktion und je näher sich die real gemessene Kurve einer solchen Stufe annähert, desto besser ist der Inverter. In einer speziellen Ausführung der Erfindung werden die Verbindungen der Formel I als Dotierstoff für organische Halbleiter in einem Inverter eingesetzt.

Die Erfindung wird anhand der folgenden nicht einschränkenden Beispiele näher erläutert.

I. Herstellung von Verbindungen der Formel I

Die Herstellung der Verbindungen der Beispiele 1-7 erfolgte wie in Angew. Chem. 2007, 119, 3824 - 3827 beschrieben.

Beispiel 1:

Verbindung der Formel I, worin $R^1$ und $R^2$ für 4-Methoxyphenyl; $R^3$ und $R^6$ für Cyano; $R^4$ und $R^5$ für 6-tert-Butyl-2-chinolyl und $R^7$ und $R^8$ für Wasserstoff stehen (Verbindung 1).

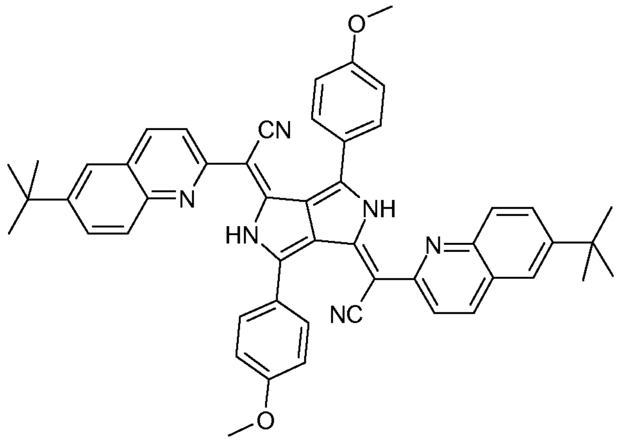

Schmelzpunkt. 392 °C, Zersetzung ab 392 °C

Absortionswerte (Abs)- und Photolumineszenzwerte (PL) von Verbindung 1 gelöst in Dichlormethan (DCM) und als 30 nm Dünnschicht auf Quarz

| | Abs (nm) | PL (nm) | |
|---|---|---|---|
| in DCM | 731 | 808 | ε = 75529 L / mol• cm |
| 30 nm Schicht | 764 | -- | ODmax= 0,33 |
| ODmax = maximale optische Dichte<br>ε = Extinktionskoeffizient | | | |

Beispiel 2:

Verbindung der Formel I, worin $R^1$ und $R^2$ für 4-Methoxyphenyl; $R^3$ und $R^6$ für Cyano; $R^4$ und $R^5$ für 6-tert-Butyl-2-chinolyl und $R^7$ und $R^8$ für $B(Phenyl)_2$ stehen (Verbindung 2).

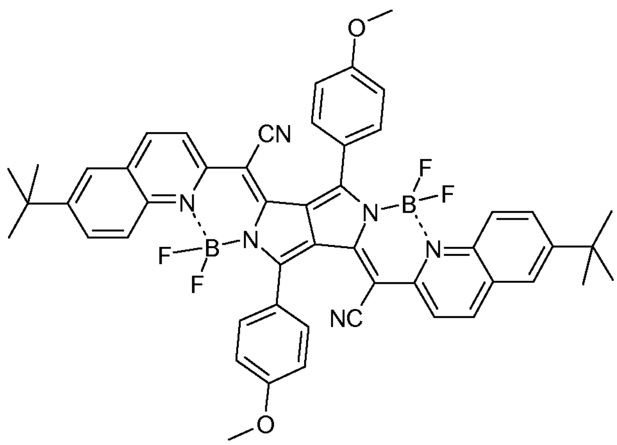

Schmelzpunkt > 400 °C, Zersetzung > 400 °C

Absortionswerte (Abs)- und Photolumineszenzwerte (PL) von Verbindung 1 gelöst in Dichlormethan (DCM) und als 30 nm Dünnschicht auf Quarz

| | Abs (nm) | PL (nm) | |
|---|---|---|---|
| in DCM | 753 | 779 | ε = 219392 L / mol• cm |
| 30 nm Schicht | 805 | 833 | ODmax= 0,49 |
| ODmax = maximale optische Dichte<br>ε = Extinktionskoeffizient | | | |

Beispiel 3:

Verbindung der Formel I, worin $R^1$ und $R^2$ für 4-Octyloxyphenyl; $R^3$ und $R^6$ für Cyano; $R^4$ und $R^5$ für 6-tert-Butyl-2-chinolyl und $R^7$ und $R^8$ für Wasserstoff stehen (Verbindung 3).

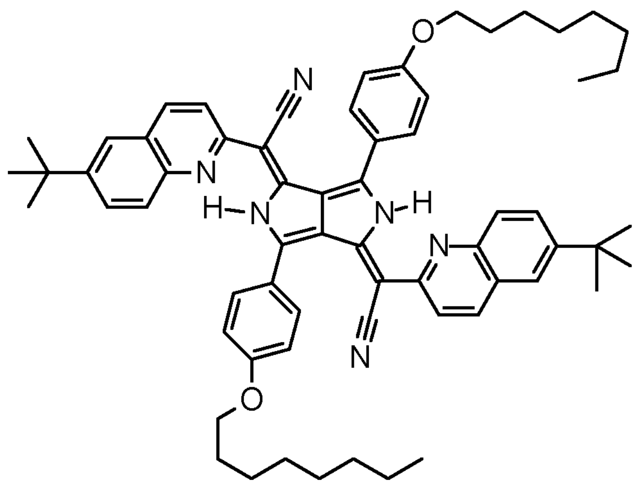

Schmelzpunkt: 269 °C, Zersetzung ab 269 °C

Beispiel 4:

Verbindung der Formel I, worin $R^1$ und $R^2$ für 4-Octyloxyphenyl; $R^3$ und $R^6$ für Cyano; $R^4$ und $R^5$ für 6-tert-Butyl-2-chinolyl und $R^7$ und $R^8$ für B(Phenyl)$_2$ stehen (Verbindung 4).

Schmelzpunkt: 311 °C, Zersetzung ab 311 °C

Beispiel 5:

Verbindung der Formel I, worin $R^1$ und $R^2$ für 4-Octyloxyphenyl; $R^3$ und $R^6$ für Cyano; $R^4$ und $R^5$ für 6-tert-Butyl-2-chinolyl und $R^7$ und $R^8$ für $BF_2$ stehen (Verbindung 5). Schmelzpunkt: 310 °C, Zersetzung 369 °C

Beispiel 6:

Verbindung der Formel I, worin $R^1$ und $R^2$ für 4-Octyloxyphenyl; $R^3$ und $R^6$ für Cyano; $R^4$ und $R^5$ für 5-tert-Butyl-1,3-benzoxazol-2-yl und $R^7$ und $R^8$ für $BF_2$ stehen (Verbindung 6).

Schmelzpunkt: 343 °C, Zersetzung ab 362 °C

Beispiel 7:

Verbindung der Formel I, worin $R^1$ und $R^2$ für 4-Octyloxyphenyl; $R^3$ und $R^6$ für Cyano; $R^4$ und $R^5$ für 5-tert-Butyl-1,3-benzoxazol-2-yl und $R^7$ und $R^8$ für $B(Phenyl)_2$ stehen (Verbindung 7)

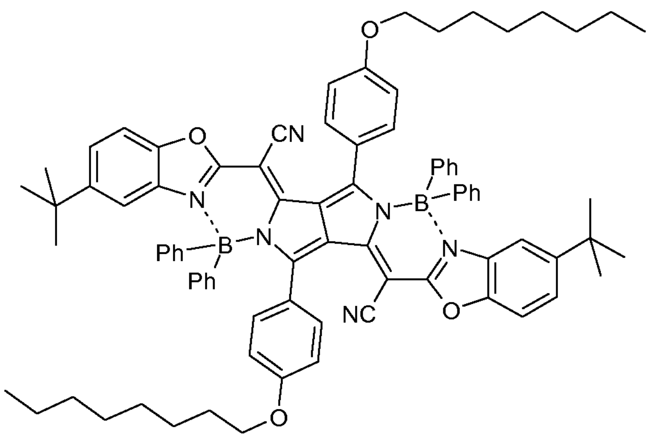

Schmelzpunkt: 322 °C, Zersetzung ab 322 °C

II. Herstellung der Zellen

Herstellung der Zellen:

Die Verbindungen 1 bis 7 wurden entweder wie aus der Synthese erhalten oder im nachgereinigten Zustand verwendet.

Substrat: ITO wurde auf das Glassubstrat in einer Dicke von 100 nm aufgesputtert. Der spezifische Widerstand betrug 30 Ohm/sq.

Es wurden Bilayer-Zellen (Zellen vom Zweischichtaufbau) und Bulk-Heterojunction-Zellen (BHJ-Zelle) im Hochvakuum (Druck im Bereich von $10^{-5}$ bis $10^{-8}$ mbar) hergestellt.

Bilayer-Zelle: (ITO / $C_{60}$/ Verbindung der Formel I / nicht-dotierte Löchertransportschicht/p-dotierte Löchertransportschicht / Au):

Die Bilayer-Zelle wurde durch zeitlich aufeinanderfolgende Abscheidung einer erfindungsgemäß eingesetzten Verbindung der Formel I und $C_{60}$ auf ITO-Substrat hergestellt. Die Abscheidungsrate betrug für beide Schichten 0,2 Å/Sekunde. Die Verdampfungstemperaturen Tv der Verbindungen der Formel I sind in der folgenden Tabelle 1 wiedergegeben. Nachfolgend wurde eine 5 nm dicke, nicht dotierte Löchertransportschicht aufgebracht. Nachdem die p-dotierte Löchertransportschicht (in allen Zellen: Dotierung mit NDP9, Novaled AG) (Schichtdicke 50 nm) aufgebracht worden war, wurde abschließend eine 50 nm dicke Au-Schicht als Topelektrode aufgedampft. Die Zelle hatte eine Fläche von 6,45 $mm^2$.

Löchertransportschicht in Bilayer-Zellen mit Verbindungen aus den Beispielen 1, 2, 3, 4, 5 oder 6: DiNPB. Löchertransportschicht in Bilayer-Zellen mit der Verbindung aus Beispiel 7: BPAPF.

BHJ-Zelle: (ITO / $C_{60}$/ Verbindung der Formel I:C60 - gewichtsmäßig 2:1 / BPAPF / BPAPF dotiert mit NDP9 / Au): Zur Herstellung der BHJ-Zelle (wurde zunächst eine 15 nm dicke $C_{60}$ Schicht auf ITO abgeschieden. Dann wurde eine

erfindungsgemäß eingesetzte Verbindung der Formel I: (Verbindung aus Beispiel 7) und $C_{60}$ gemeinsam verdampft und auf der $C_{60}$-Schicht mit Abscheidungsraten von insgesamt 0,3 Å/Sekunden abgeschieden, so dass in der gemischten aktiven Schicht ein Massenverhältnis von 2 : 1 vorlag. Darauf folgten analog zu der Bilayer Zelle eine 5 nm dicke undotierte Löchertransportschicht (BPAPF), eine 50 nm dicke dotierte Löchertransportschicht (BPAPF dotiert mit NDP9; NDP9 ist käuflich von Novaled AG, Dresden) und ein 50 nm dicker Au-Top-Kontakt. Die Kenndaten und die Verdampfungstemperatur Tv sind in Tabelle 2 angegeben.

Messungen:

Als Strahlungsquelle wurde ein Sonnensimulator (Hoenle SOL 1200) verwendet. Die Strahlungsleistung $Ir_{rad}$ ist in den Tabellen 1 und 2 angegeben

Tabelle 1: Kenndaten der Bilayer-Zelle, die Verdampfungstemperaturen Tv sind ebenfalls aufgeführt:

| Verbindung I aus | $T_V$ [°C] | Verb. I Dicke [nm] | $Ir_{rad}$ [mW/cm$^2$] | $C_{60}$ Dicke [nm] | $V_{OC}$ [mV] | $I_{SC}$ [mA/cm$^2$] | FF [%] | η [%] | max. EQE |
|---|---|---|---|---|---|---|---|---|---|
| Bsp.1 | 330 | 6 | 110 | 15 | 0,53 | 2,9 | 26,8 | 0,4 | 0,15 |
| Bsp.1 | 330 | 10 | 110 | 15 | 0,51 | 2,5 | 19,6 | 0,2 | 0,12 |
| Bsp.2 | 330-420 | 6 | 109 | 15 | 0,84 | 2,4 | 31,3 | 0,6 | 0,30 |
| Bsp.2 | 330-420 | 10 | 108 | 15 | 0,82 | 2,1 | 19,0 | 0,3 | 0,29 |
| Bsp.3 | 290-330 | 6 | 98 | 15 | 0,56 | 2,7 | 56,9 | 0,9 | 0,15 |
| Bsp.3 | 290-330 | 10 | 98 | 15 | 0,58 | 2,6 | 54,7 | 0,8 | 0,15 |
| Bsp.4 | 310-340 | 6 | 99 | 15 | 0,69 | 3,7 | 23,8 | 0,6 | 0,38 |
| Bsp.4 | 310-340 | 10 | 99 | 15 | 0,69 | 1,0 | 12,5 | 0,1 | 0,21 |
| Bsp.5 | 310-330 | 6 | 105 | 15 | 0,91 | 2,4 | 40,1 | 0,8 | 0,29 |
| Bsp.5 | 310-330 | 10 | 105 | 15 | 0,91 | 2,3 | 38,1 | 0,8 | 0,3 |
| Bsp.6 | 250-290 | 6 | 105 | 15 | 0,92 | 0,9 | 22,5 | 0,2 | 0,09 |
| Bsp.6 | 250-290 | 10 | 105 | 15 | 0,92 | 0,8 | 21,3 | 0,2 | 0,08 |
| Bsp.7 | 280-290 | 6 | 106 | 15 | 0,95 | 3,6 | 68,6 | 2,2 | 0,40 |
| Bsp.7 | 280-290 | 10 | 106 | 15 | 0,95 | 4,1 | 58,5 | 2,2 | 0,53 |

Tabelle 2: BHJ-Zelle:

| Verbindung I aus | $T_V$ [°C] | $Ir_{rad}$ [mW/cm$^2$] | BHJ-Dicke [nm] | $C_{60}$ Dicke [nm] | $V_{OC}$ [mV] | $I_{SC}$ [mA/cm$^2$] | FF [%] | η [%] | max EQE |
|---|---|---|---|---|---|---|---|---|---|
| Beispiel 7 | 280-290 | 112 | 10 | 15 | 0,97 | 3,1 | 54,0 | 1,45 | 0,30 |
| Beispiel 7 | 280-290 | 113 | 20 | 15 | 0,97 | 2,8 | 35,3 | 0,85 | 0,31 |

η Wirkungsgrad
FF Füllfaktor
$I_{sc}$ Kurzschlussstromstärke
$V_{oc}$ Leerlaufspannung
$Ir_{rad}$ Strahlungsleistung
max EQE maximale externe Quanteneffizienz

## Patentansprüche

1. Vorrichtung der organischen Elektronik, umfassend mindestens zwei Elektroden und mindestens eine zwischen

den Elektroden liegende organische Schicht, wobei die organische Schicht wenigstens eine Pyrrolopyrrol-Cyanin-Verbindung der Formel I enthält

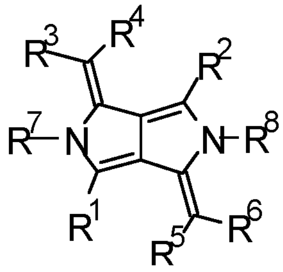

(I)

worin

$R^1$ und $R^2$ unabhängig voneinander ausgewählt sind unter Wasserstoff, $C_1$-$C_{20}$-Alkyl, $C_1$-$C_{20}$-Alkoxy, $C_3$-$C_{15}$-Cycloalkyl, $C_3$-$C_{15}$-Cycloalkenyl, $C_3$-$C_{15}$-Cycloalkyloxy, 3- bis 15-gliedrigem gesättigten oder partiell ungesättigten Heterocyclus, 3- bis 15-gliedrigem gesättigten oder partiell ungesättigten Heterocyclyloxy, $C_6$-$C_{14}$-Aryl, $C_6$-$C_{14}$-Aryloxy, 5- bis 14-gliedriges Heteroaryl und 5- bis 14-gliedriges Heteroaryloxy, wobei die acht letztgenannten Reste unsubstituiert sind oder einen oder mehrere Reste $R^{1a}$ tragen, wobei jedes $R^{1a}$ unabhängig voneinander für $NR^{1b}R^{1c}$, $C_1$-$C_{20}$-Alkyl, $C_2$-$C_{20}$-

Alkenyl, $C_2$-$C_{20}$-Alkinyl, $C_1$-$C_{20}$-Alkoxy, $C_1$-$C_{20}$-Alkoxy-$C_1$-$C_{20}$-alkyl, $C_1$-$C_{20}$-Alkoxy, das im Alkylteil ein- oder mehrfach durch nicht benachbarte Sauerstoffatome unterbrochen ist, $C_1$-$C_{20}$-Alkylthio, $C_1$-$C_{20}$-Haloalkyl, $C_1$-$C_{20}$-Haloalkoxy, $C_2$-$C_{20}$-Haloalkenyl, $C_2$-$C_{20}$-Haloalkinyl, $C_2$-$C_{20}$-Alkenyloxy, $C_2$-$C_{20}$-Alkinyloxy, $C_6$-$C_{14}$-Aryl-$C_1$-$C_{10}$-alkyl, $C_6$-$C_{14}$-Aryl-$C_2$-$C_{10}$-alkenyl oder $C_6$-$C_{14}$-Aryl-$C_2$-$C_{10}$-alkinyl steht,
wobei
$R^{1b}$ und $R^{1c}$ unabhängig voneinander für $C_1$-$C_{20}$-Alkyl oder $C_6$-$C_{14}$-Aryl stehen;
wobei die aliphatischen oder aromatischen Gruppen in der vorgenannten Gruppe $R^{1a}$ ihrerseits eine, zwei oder drei Gruppen $NR^{1b}R^{1c}$ tragen können

einer der Reste $R^3$ oder $R^4$ für 5- bis 14-gliedriges Heteroaryl steht, das unsubstituiert ist oder einen oder mehrere Reste $R^x$ trägt, und der andere Rest $R^3$ oder $R^4$ für eine elektronenziehende Gruppe $E^1$ steht, wobei

jedes $R^x$ unabhängig voneinander für Halogen, $C_1$-$C_{20}$-Alkyl, $C_2$-$C_{20}$-Alkenyl, $C_2$-$C_{20}$-Alkinyl, $C_1$-$C_{20}$-Alkoxy, $C_1$-$C_{20}$-Alkylthio, $C_1$-$C_{20}$-Haloalkyl, $C_1$-$C_{20}$-Haloalkoxy, $C_2$-$C_{20}$-Haloalkenyl, $C_2$-$C_{20}$-Haloalkinyl, $C_2$-$C_{20}$-Alkenyloxy, $C_2$-$C_{20}$-Alkinyloxy, $C_6$-$C_{14}$-Aryl, $C_6$-$C_{14}$-Aryl-$C_1$-$C_{10}$-alkyl, $C_6$-$C_{14}$-Aryl-$C_2$-$C_{10}$-alkenyl oder $C_6$-$C_{14}$-Aryl-$C_2$-$C_{10}$-alkinyl steht; und
$E^1$ für Cyano, Nitro, $SO_3H$, CHO, $COOR^{E1}$, $COR^{E1}$, $N(R^{E2})_3{}^+X^-$ steht, wobei

$R^{E1}$ für Wasserstoff oder $C_1$-$C_{10}$-Alkyl steht;
jedes $R^{E2}$ unabhängig voneinander für Wasserstoff oder $C_1$-$C_{10}$-Alkyl steht,

$X^-$ für ein Anionäquivalent steht;

einer der Reste $R^5$ oder $R^6$ für 5- bis 14-gliedriges Heteroaryl steht, das unsubstituiert ist oder einen oder mehrere Reste $R^x$ trägt, und der andere Rest $R^5$ oder $R^6$ für eine elektronenziehende Gruppe $E^2$ steht, wobei $E^2$ wie $E^1$ definiert ist;
$R^7$ und $R^8$, unabhängig voneinander, ausgewählt sind unter Wasserstoff, $C_1$-$C_4$-Alkyl und $B(R^9R^{10})$, wobei

$R^9$ für Halogen, $C_1$-$C_{10}$-Alkyl, $C_3$-$C_{10}$-Cycloalkyl, $C_3$-$C_{15}$-Cycloalkenyl oder $C_6$-$C_{14}$-Aryl steht, wobei die drei letztgenannten zyklischen Reste unsubstituiert sind oder einen oder mehrere Reste $R^{9a}$ tragen, wobei $R^{9a}$ wie $R^x$ definiert ist; und
$R^{10}$ wie $R^9$ definiert ist.

**2.** Vorrichtung der organischen Elektronik nach Anspruch 1, ausgewählt unter einer organischen Solarzelle, organischen Elektrolumineszenzvorrichtung, einem Photodetektor und einem organischen Feldeffekttransistor.

3. Vorrichtung der organischen Elektronik nach Anspruch 1 oder 2, worin in der Verbindung der Formel I $R^1$ und $R^2$ unabhängig voneinander ausgewählt sind unter $C_6$-$C_{10}$-Aryl und 5- bis 14-gliedrigem Heteroaryl, wobei Aryl und Heteroaryl in den zwei letztgenannten Resten unsubstituiert sind oder 1, 2 oder 3 Reste $R^{1a}$ aufweisen und Heteroaryl 1, 2 oder 3 unter N, O und S ausgewählte Heteroatome als Ringglieder enthält.

4. Vorrichtung der organischen Elektronik nach Anspruch 3, worin in der Verbindung der Formel I $R^1$ und $R^2$ unabhängig voneinander ausgewählt sind unter Phenyl und Phenyl, das einen Substituenten $R^{1a}$ trägt.

5. Vorrichtung der organischen Elektronik nach einem der vorhergehenden Ansprüche, worin in der Verbindung der Formel I einer der Reste $R^3$ oder $R^4$ und einer der Reste $R^5$ oder $R^6$ unabhängig voneinander ausgewählt sind unter 5- oder 6-gliedrigem monozyklischen Heteroaryl und 9- oder 10-gliedrigem bizyklischen Heteroaryl, wobei 5- oder 6-gliedriges monozyklisches Heteroaryl und 9- oder 10-gliedriges bizyklisches Heteroaryl unsubstituiert sind oder einen oder mehrere Reste $R^x$ aufweisen und wobei 5- oder 6-gliedriges monozyklisches Heteroaryl und 9- oder 10 gliedriges bizyklisches Heteroaryl als Ringglieder 1, 2, 3 oder 4 unter O, S und N ausgewählte Heteroatome aufweisen und wenigstens ein Heteroatom im monozyklischem oder bizyklischem Heteroaryl sich in alpha-Position zur Bindungsstelle an das Pyrrolopyrrole-Grundgerüst befindet.

6. Vorrichtung der organischen Elektronik nach Anspruch 5, worin in der Verbindung der Formel I einer der Reste $R^3$ oder $R^4$ und einer der Reste $R^5$ oder $R^6$ unabhängig voneinander ausgewählt sind unter den Resten der Formeln Het-1, Het-2, Het-3, Het-4, Het-5 und Het-6

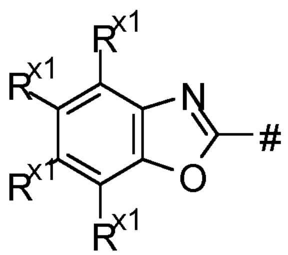

Het-1

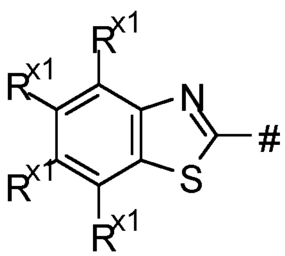

Het-2

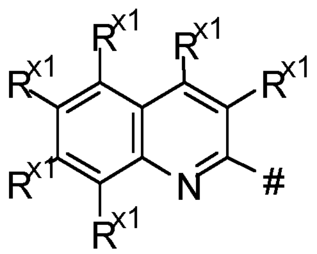

Het-3

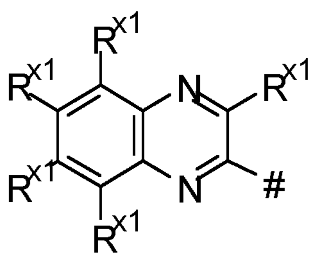

Het-4

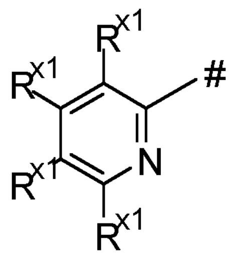

Het-5

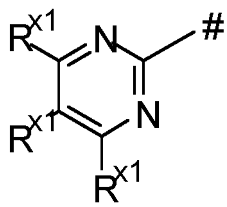

Het-6

worin

# für die Bindungsstelle zu dem Pyrrolopyrrol-Grundgerüst steht; und
jedes $R^{x1}$ unabhängig voneinander für Wasserstoff steht oder eine der für $R^x$ genannten Bedeutungen aufweist.

7. Vorrichtung der organischen Elektronik nach Anspruch 6, worin in der Verbindung der Formel I einer der Reste $R^3$ oder $R^4$ und einer der Reste $R^5$ oder $R^6$ unabhängig voneinander ausgewählt sind Cyano oder Nitro.

8. Vorrichtung der organischen Elektronik nach einem der vorhergehenden Ansprüche, worin in der Verbindung der Formel I die Reste $R^3$ und $R^6$ und die Reste $R^4$ und $R^5$ die gleiche Bedeutung aufweisen.

9. Vorrichtung der organischen Elektronik nach einem der vorhergehenden Ansprüche, worin die Reste $R^7$ und $R^8$

unabhängig voneinander ausgewählt sind unter Wasserstoff, $BF_2$, $B(C_1\text{-}C_8\text{-Alkyl})_2$ und $B(\text{Phenyl})_2$.

**10.** Vorrichtung der organischen Elektronik nach einem der vorhergehenden Ansprüche, worin in der Verbindung der Formel I
$R^1$ und $R^2$ identisch sind und ausgewählt sind unter Phenyl und Phenyl, das einen Substituenten $R^{1x}$ trägt;
$R^3$ und $R^6$ für Cyano stehen;
$R^4$ und $R^5$ unabhängig voneinander für einen Rest ausgewählt unter Het-1, Het-2, Het-3, Het-4, Het-5 und Het-6 stehen; und
$R^7$ und $R^8$ unabhängig voneinander für einen Rest ausgewählt unter Wasserstoff, $BF_2$ und $B(\text{Phenyl})_2$ stehen.

**11.** Vorrichtung der organischen Elektronik nach einem der vorhergehenden Ansprüche, die eine organische Solarzelle mit photoaktiven Donor-Akzeptor-Übergängen ist, vorzugsweise in Form einer bulk heterojunction.

**12.** Vorrichtung der organischen Elektronik nach Anspruch 11, worin die organische Solarzelle in Form einer einzelnen Zelle, einer Tandemzelle oder einer Mehrfachsolarzelle ist.

**13.** Organische Solarzelle mit einem photoaktiven Bereich, der wenigstens ein organisches Donormaterial in Kontakt mit wenigstens einem organischen Akzeptormaterial aufweist, wobei das Donormaterial und das Akzeptormaterial einen Donor-Akzeptor-Heteroübergang ausbilden und wobei der photoaktive Bereich wenigstens eine Verbindung der Formel I, wie in einem der Ansprüche 1 bis 10 definiert, enthält.

**14.** Organischer Feldeffekttransistor, enthaltend ein Substrat mit wenigstens einer Gate-Struktur und einer Drain- und Sourceelektrode und wenigstens eine Verbindung der Formel I, wie in einem der Ansprüche 1 bis 10 definiert.

**15.** Substrat mit einer Vielzahl von organischen Feldeffekttransistoren, wobei zumindest ein Teil der Feldeffekttransistoren wenigstens eine Verbindung der Formel I, wie in einem der Ansprüche 1 bis 10 definiert, enthält.

**16.** Halbleiterbaustein, umfassend wenigstens ein Substrat, wie in Anspruch 15 definiert.

**17.** Organische Leuchtdiode, enthaltend eine Anode, eine Kathode und eine zwischen der Anode und Kathode angeordnete Licht emittierende Schicht sowie gegebenenfalls mindestens eine weitere Schicht ausgewählt aus der Gruppe bestehend aus einer Blockschicht für Elektronen/Excitonen, mindestens einer Blockschicht für Löcher/Excitonen, mindestens einer Loch-Injektionsschicht, mindestens einer Lochleiterschicht, mindestens einer Elektroneninjektionsschicht und mindestens einer Elektronenleiterschicht, wobei mindestens eine Verbindung der allgemeinen Formel I, wie in einem der Ansprüche 1 bis 10 definiert, in der Licht emittierenden Schicht und/oder in der mindestens einen weiteren Schicht enthalten ist.

## Claims

**1.** An organic electronic device comprising at least two electrodes and at least one organic layer located between the electrodes, wherein the organic layer contains at least one pyrrolopyrrole-cyanine compound of the formula I.

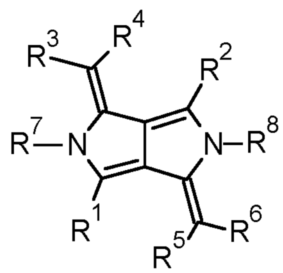

(I)

wherein

$R^1$ and $R^2$ are independently selected from hydrogen, C1-C20 alkyl, C1-C20 alkoxy, C3-C15 cycloalkyl, C3-C15 cycloalkenyl, C3-C15 cycloalkyloxy, 3- to 15-membered saturated or partially unsaturated Heterocycle, 3- to 15-membered saturated or partially unsaturated heterocyclyloxy, C6-C14-aryl, C6-C14-aryloxy, 5- to 14-membered heteroaryl and 5- to 14-membered heteroaryloxy, wherein the eight latter radicals are unsubstituted or carry one or more radicals $R^{1a}$, wherein each $R^{1a}$ is independently $NR^{1b}R^{1c}$, C1-C20 alkyl, C2-C20 alkenyl, C2-C20 alkynyl, C1-C20 alkoxy, C1-C20 alkoxy-C1-C20 alkyl, C1-C20-alkoxy, which in the alkyl part is interrupted one or more times by nonadjacent oxygen atoms, C1-C20-alkylthio, C1-C20-haloalkyl, C1-C20-haloalkoxy, C2-C20-haloalkenyl, C2-C20-haloalkynyl, C2-C20-alkenyloxy, C2-C20-alkynyloxy, C6-C14-aryl-C1-C10-alkyl, C6-C14-aryl-C2-C10-alkenyl or C6-C14-aryl-C2-C10-alkynyl, wherein $R^{1b}$ and $R^{1c}$ are independently C1-C20 alkyl or C6-C14 aryl; where the aliphatic or aromatic groups in the abovementioned group $R^{1a}$ in turn can carry one, two or three groups $NR^{1b}R^{1c}$

one of $R^3$ or $R^4$ is 5- to 14-membered heteroaryl which is unsubstituted or carries one or more $R^x$ and the other $R^3$ or $R^4$ is an electron withdrawing group $E^1$, wherein

each $R^x$ is independently halogen, C1-C20 alkyl, C2-C20 alkenyl, C2-C20 alkynyl, C1-C20 alkoxy, C1-C20 alkylthio, C1-C20 haloalkyl, C1-C20 haloalkoxy, C2 -C20 haloalkenyl, C2-C20 haloalkynyl, C2-C20 alkenyloxy, C2-C20 alkynyloxy, C6-C14 aryl, C6-C14 aryl C1-C10 alkyl, C6-C14 aryl C2-C10 alkenyl or C6-C14 aryl-C2-C10 alkynyl; and E1 is cyano, nitro, $SO_3H$, CHO, $COOR^{E1}$, $COR^{E1}$, $N(R^{E2})_3$ + X-, where $R^{E1}$ is hydrogen or C1-C10 alkyl; each $R^{E2}$ is independently hydrogen or C1-C10 alkyl, X- is an anion equivalent; one of $R^5$ or $R^6$ is 5- to 14-membered heteroaryl which is unsubstituted or carries one or more $R^x$ and the other $R^5$ or $R^6$ is an electron-withdrawing group $E^2$ wherein $E^2$ is defined as $E^1$ is;

$R^7$ and $R^8$, independently of one another, are selected from hydrogen, C1-C4-alkyl and B ($R^9R^{10}$), where $R^9$ is halogen, C1-C10-alkyl, C3-C10-cycloalkyl, C3-C15-cycloalkenyl or C6-C14- Aryl, wherein the three last-mentioned cyclic radicals are unsubstituted or carry one or more radicals $R^{9a}$, wherein $R^{9a}$ is defined as $R^x$; and $R^{10}$ is defined as $R^9$.

**2.** An organic electronic device according to claim 1, selected from an organic solar cell, organic electroluminescent device, a photodetector and an organic field effect transistor.

**3.** An organic electronic device according to claim 1 or 2, wherein in the compound of formula I $R^1$ and $R^2$ are independently selected from C6-C10 aryl and 5- to 14-membered heteroaryl, wherein aryl and heteroaryl in the two latter radicals are unsubstituted or have 1, 2 or 3 radicals $R^{1a}$ and heteroaryl contains 1, 2 or 3 heteroatoms selected from N, O and S as ring members.

**4.** The organic electronic device according to claim 3, wherein in the compound of formula I, $R^1$ and $R^2$ are independently selected from phenyl and phenyl bearing a substituent $R^{1a}$.

**5.** An organic electronic device according to any one of the preceding claims, wherein in the compound of formula I one of $R^3$ or $R^4$ and one of $R^5$ or $R^6$ are independently selected from 5- or 6-membered monocyclic heteroaryl and 9 or 10-membered bicyclic heteroaryl, wherein 5- or 6-membered monocyclic heteroaryl and 9- or 10-membered bicyclic heteroaryl are unsubstituted or have one or more $R^x$ and wherein 5- or 6-membered monocyclic heteroaryl and 9- or 10-membered bicyclic heteroaryl as ring members 1, 2, 3 or 4 have heteroatoms selected from O, S and N and at least one heteroatom in the monocyclic or bicyclic heteroaryl is in the alpha position to the point of attachment to the pyrrolopyrrole skelleton.

**6.** An organic electronic device according to claim 5, wherein in the compound of the formula I one of the radicals $R^3$ or $R^4$ and one of the radicals $R^5$ or $R^6$ are independently selected from the radicals of the formulas Het-1, Het-2, Het-3 , Het-4, Het-5 and Het-6

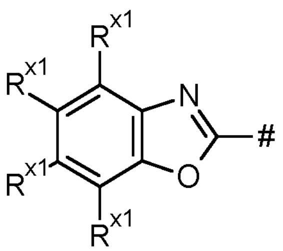

Het-1

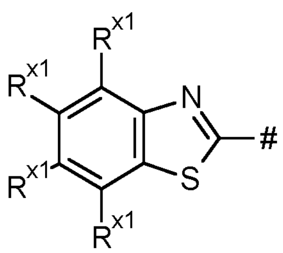

Het-2

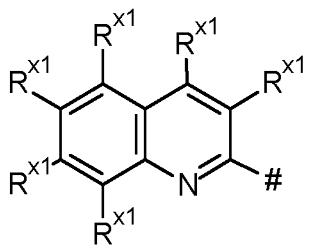

Het-3

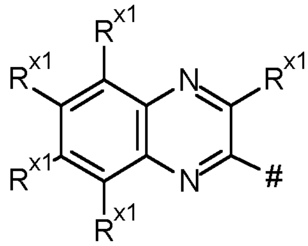
Het-4

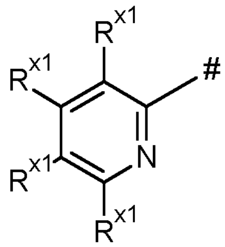
Het-5

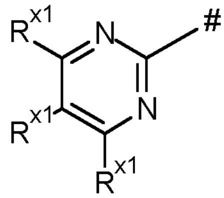
Het-6

wherein

# represents the point of attachment to the pyrrolopyrrole skeleton; and
each $R^{x1}$ is independently hydrogen or has one of the meanings given for $R^x$.

**7.** An organic electronic device according to claim 6, wherein in the compound of the formula I one of the radicals $R^3$ or $R^4$ and one of the radicals $R^5$ or $R^6$ are independently selected from cyano or nitro.

**8.** An organic electronic device according to any one of the preceding claims, wherein in the compound of formula I, the radicals $R^3$ and $R^6$ and the radicals $R^4$ and $R^5$ have the same meaning.

**9.** An organic electronic device according to any one of the preceding claims, wherein $R^7$ and $R^8$ are independently selected from hydrogen, $BF_2$, B (C1-C8 alkyl)$_2$ and B (phenyl)$_2$.

**10.** An organic electronics device according to any one of the preceding claims, wherein in the compound of formula I

$R^1$ and $R^2$ are identical and are selected from phenyl and phenyl bearing a substituent $R^{1x}$;
$R^3$ and $R^6$ are cyano;
$R^4$ and $R^5$ independently represent a group selected from Het-1, Het2, Het-3, Het-4, Het-5 and Het-6; and
$R^7$ and $R^8$ independently represent a radical selected from among hydrogen, $BF_2$ and B (phenyl)$_2$.

**11.** An organic electronic device according to any one of the preceding claims, which is an organic solar cell having photoactive donor-acceptor transitions, preferably in the form of a bulk heterojunction.

**12.** The organic electronic device according to claim 11, wherein said organic solar cell is in the form of a single cell, a tandem cell or a multiple solar cell.

**13.** An organic solar cell having a photoactive region comprising at least one organic donor material in contact with at least one organic acceptor material, wherein the donor material and the acceptor material form a donor-acceptor heterojunction and wherein the photoactive region comprises at least one compound of the formula I as defined in any one of claims 1 to 10.

**14.** An organic field effect transistor comprising a substrate having at least one gate structure and a drain and source electrode and at least one compound of the formula I as defined in any one of claims 1 to 10.

**15.** A substrate having a multiplicity of organic field-effect transistors, wherein at least part of the field-effect transistors contains at least one compound of the formula I as defined in one of claims 1 to 10.

**16.** A semiconductor device comprising at least one substrate as defined in claim 15.

**17.** Organic light-emitting diode, comprising an anode, a cathode and a light-emitting layer arranged between the anode and cathode and optionally at least one further layer selected from the group consisting of a block layer for electrons / excitons, at least one block layer for holes Excitons, at least one hole injection layer, at least one hole conductor layer, at least one electron injection layer and at least one electron conductor layer, wherein at least one compound of general formula I, as defined in any one of claims 1 to 10, in the light-emitting Layer and / or contained in the at least one further layer.

**Revendications**

1. Dispositif électronique organique comprenant au moins deux électrodes et au moins une couche organique entre les électrodes, la couche organique contenant au moins un composé pyrrolopyrrole-cyanine de formule I.

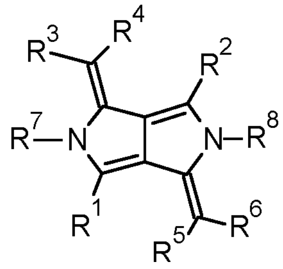

(I)

où

$R^1$ et $R^2$ sont indépendamment choisis parmi hydrogène, alkyle en C1-C20, alcoxy en C1-C20, cycloalkyle en C3-C15, cycloalcényle en C3-C15, cycloalkyloxy en C3-C15, saturé ou partiellement insaturé Hétérocycle, hétérocyclyloxy saturé ou partiellement insaturé de 3 à 15 chaînons, aryle en C6-C14, aryloxy en C6-C14, hétéroaryle de 5 à 14 chaînons et hétéroaryloxy de 5 à 14 chaînons, où les huit derniers radicaux ne sont pas substitués ou portent un ou plusieurs radicaux $R^{1a}$,
dans lesquels chaque $R^{1a}$ représente indépendamment un groupe $NR^{1b}R^{1c}$, alkyle en C1 à C20, alcényle en C2 à C20, alcynyle en C2 à C20, alcoxy en C1 à C20, alcoxy en C1 à C20, alkyle en C1 à C20 Alcoxy en C1-C20, qui dans le fragment alkyle est interrompu une ou plusieurs fois par des atomes d'oxygène non adjacents, alkylthio en C1-C20, halogénalkyle en C1-C20, halogénalkoxy en C2-C20, halogénoalkynyle en C2-C20, Alcényloxy en C2 à C20, alcynyloxy en C2 à C20, aryle en C6 à C14, (alkyle en C1 à C10), aryl en C6 à C14 ou alcényle en C6 à C14,
dans lesquels $R^{1b}$ et $R^{1c}$ sont indépendamment un groupe alkyle en C1 à C20 ou aryle en C6 à C14;
où les groupes aliphatiques ou aromatiques du groupe susmentionné $R^{1a}$ peuvent à leur tour porter un, deux ou trois groupes $NR^{1b}R^{1c}$ l'un de $R^3$ ou $R^4$ est un hétéroaryle de 5 à 14 chaînons qui est non substitué ou qui porte un ou plusieurs $R^x$ et l'autre $R^3$ ou $R^4$ est un groupe électro-attracteur $E^1$, dans lequel
chaque $R^x$ est indépendamment un atome d'halogène, un groupe alkyle en C1 à C20, alcényle en C2 à C20, alcynyle en C2 à C20, alcoxy en C1 à C20, alkylthio en C1 à C20, halogénalkyle en C1 à C20, halogénalkoxy en C1 à C20, C2 Halo-génoalcényle en C20 à C, halogénoalcynyle en C2 à C20, alcényloxy en C2 à C20, alcynyloxy en C2 à C20, aryle en C6 à C14, aryle en C6 à C14, aryle en C6 à C14, aryl en C2 à C10 alcényle ou aryle en C6 à C14 - alcynyle en C2 à C10; et $E^1$ est un groupe cyano, nitro, $SO_3H$, CHO, $COOR^{E1}$, $COR^{E1}$, $N(R^{E2})_3$ + X-, où $R^{E1}$ est un atome d'hydrogène ou un groupe alkyle en C1 à C10; chaque groupe $R^{E2}$ représente indépendamment un atome d'hydrogène ou un groupe alkyle en C1 à C10, X- représente un équivalent d'anion;
l'un de $R^5$ ou $R^6$ est un hétéroaryle de 5 à 14 chaînons qui est non substitué ou qui porte un ou plusieurs $R^x$ et l'autre R5 ou R6 est un groupe attracteur d'électrons $E^2$ où $E^2$ est défini par $E^1$ est;
$R^7$ et $R^8$, indépendamment l'un de l'autre, sont choisis parmi l'hydrogène, les groupes alkyle en C1 à C4 et B $(R^9R^{10})$, où $R^9$ représente un atome d'halogène, un groupe alkyle en C1 à C10, cycloalkyle en C3 à C10, cycloalcényle en C3 à C15 ou C6 à C14. Aryle, dans lequel les trois derniers radicaux cycliques mentionnés sont non substitués ou portent un ou plusieurs radicaux $R^{9a}$, dans lesquels R9a est défini comme $R^x$; et $R^{10}$ est défini comme $R^9$.

2. Dispositif électronique organique selon la revendication 1, choisi parmi une cellule solaire organique, un dispositif électroluminescent organique, un photodétecteur et un transistor à effet de champ organique.

3. Dispositif électronique organique selon la revendication 1 ou 2, dans lequel, dans le composé de formule I, $R^1$ et $R^2$ sont indépendamment choisis parmi aryle en C6-C10 et hétéroaryle de 5 à 14 chaînons, dans lequel aryle et hétéroaryle les derniers radicaux sont non substitués ou ont 1, 2 ou 3 radicaux $R^1$ a et hétéroaryle contient 1, 2 ou 3 hétéroatomes choisis parmi N, O et S en tant que chaînons du cycle.

4. Diposotifde l'électronique organique selon la revendication 3, dans lequel, dans le composé de formule I $R^1$ et $R^2$ sont choisis indépendamment parmi un groupe phényle et un groupe phényle portant un substituant $R^{1a}$.

5. Dispositif électronique organique selon l'une quelconque des revendications précédentes, dans lequel, dans le composé de formule I, l'un de $R^3$ ou $R^4$ et l'un de $R^5$ ou $R^6$ sont indépendamment choisis parmi un hétéroaryle monocyclique à 5 ou 6 chaînons et 9 ou 10. membre hétéroaryle bicyclique, dans lequel hétéroaryle monocyclique à 5 ou 6 chaînons et hétéroaryle bicyclique à 9 ou 10 chaînons sont non substitués ou ont un ou plusieurs $R^x$ et dans lequel hétéroaryle monocyclique à 5 ou 6 chaînons et 9 ou 10 chaînons hétéroaryle bi-cyclique en tant que chaînon 1, 2, 3 ou 4 parmi les hétéroatomes choisis parmi O, S et N et au moins un hétéroatome dans l'hétéroaryle monocyclique ou bicyclique est en position alpha jusqu'au point de fixation au squelette pyrrolopyrrole.

6. Dispositif électronique organique selon la revendication 5, dans lequel, dans le composé de formule I, l'un des radicaux $R^3$ ou $R^4$ et l'un des radicaux $R^5$ ou $R^6$ sont choisis indépendamment parmi les radicaux des formules Het-1, Het-2, Het-3, Het-4, Het-5 et Het-6

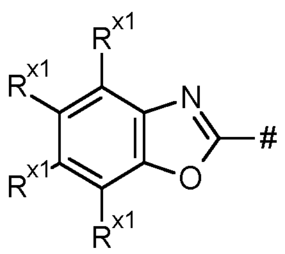

Het-1

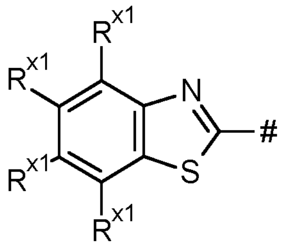

Het-2

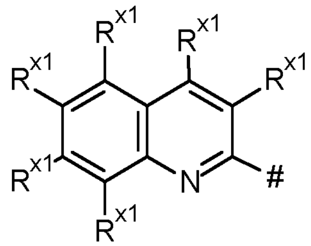

Het-3

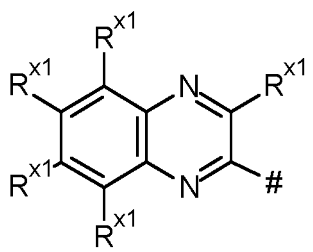

Het-4

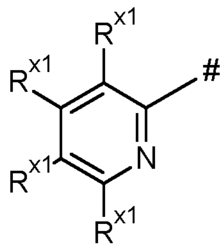

Het-5

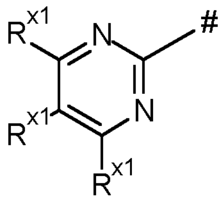

Het-6

où

# représente le point de fixation au squelette pyrrolo-pyrrole; et
chaque $R^{x1}$ est indépendamment l'hydrogène ou a l'une des significations données pour $R^x$.

7. Dispositif électronique organique selon la revendication 6, dans lequel, dans le composé de formule I, l'un des radicaux $R^3$ ou $R^4$ et l'un des radicaux $R^5$ ou $R^6$ sont choisis indépendamment parmi les radicaux cyano ou nitro.

8. Dispositif selon l'une quelconque des revendications précédentes, dans lequel, dans le composé de formule I, les radicaux $R^3$ et $R^6$ et les radicaux $R^4$ et $R^5$ ont la même signification.

9. Dispositif électronique organique selon l'une quelconque des revendications précédentes, dans lequel $R^7$ et $R^8$ sont choisis indépendamment parmi l'hydrogène, le $BF_2$, le B (alkyle en C1-C8)$_2$ et le B (phényle)$_2$.

10. Dispositif électronique organique selon l'une quelconque des revendications précédentes, dans lequel, dans le composé de formule I,

$R^1$ et $R^2$ sont identiques et sont choisis parmi phényle et phényle portant un substituant $R^{1x}$; $R^3$ et $R^6$ sont cyano;
$R^4$ et $R^5$ représentent indépendamment un groupe choisi parmi Het-1, Het-2, Het-3, Het-4, Het-5 et Het-6;
et $R^7$ et $R^8$ représentent indépendamment un radical choisi parmi hydrogène, $BF_2$ et B (phényle)$_2$.

11. Dispositif électronique organique selon l'une quelconque des revendications précédentes, qui est une cellule solaire organique ayant des transitions photoactives donneur-accepteur, de préférence sous la forme d'une hétérojonction

en masse.

**12.** Dispositif électronique organique selon la revendication 11, dans lequel ladite cellule solaire organique se présente sous la forme d'une cellule unique, d'une cellule tandem ou d'une cellule solaire multiple.

**13.** Cellule solaire organique ayant une région photoactive comprenant au moins un matériau donneur organique en contact avec au moins un matériau organique accepteur, dans laquelle le matériau donneur et le matériau accepteur forment une hétérojonction donneur-accepteur et dans laquelle la région photoactive comprend au moins un composé de formule I tel que défini dans l'une quelconque des revendications 1 à 10.

**14.** Un transistor à effet de champ organique comprenant un substrat ayant au moins une structure de grille et un drain et l'électrode de source et au moins un composé de formule I tel que défini dans l'une quelconque des revendications 1 à 10 degrés.

**15.** Substrat comportant une multiplicité de transistors organiques à effet de champ, dans lequel au moins une partie des transistors à effet de champ contient au moins un composé de formule I telle que définie dans l'une des revendications 1 à 10.

**16.** Dispositif semi-conducteur comprenant au moins un substrat tel que défini dans la revendication 15.

**17.** Diode électroluminescente organique comprenant une anode, une cathode et une couche électroluminescente disposée entre l'anode et la cathode et éventuellement au moins une autre couche choisie dans le groupe comprenant une couche de blocage pour électrons / excitons, au moins une couche de blocage pour des trous Excitons, au moins une couche d'injection de trous, au moins une couche de conducteurs de trous, au moins une couche d'injection d'électrons et au moins une couche de conducteurs d'électrons, dans lesquels au moins un composé de formule générale I, telle que définie dans l'une quelconque des revendications 1 à 10, dans le composant électroluminescent Couche et / ou contenue dans la au moins une autre couche.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2006111511 A
- WO 2007116001 A
- WO 2008145172 A
- EP 2272849 A1
- US 20050098726 A
- US 20050224905 A
- US 6451415 B
- US 20050227406 A
- US 4461922 A
- US 6198091 B
- US 6198092 B
- DE 10313232
- US 20060202195 A
- US 20070190783 A
- US 20040046182 A


### In der Beschreibung aufgeführte Nicht-Patentliteratur


- *Angew. Chem.,* 2007, vol. 119, 3824-3827
- *Angew. Chem. Int. Ed.,* 2007, vol. 46, 3750-3753
- *Angew. Chem.,* 2011, vol. 123, 1442-1445
- *Angew. Chem. Int. Ed.,* 2011, vol. 50, 1406-1409
- *Eur. J. Org. Chem.,* 2011, 3421-3429
- *Chem. Comm.,* 2010, vol. 46, 5289-5291
- *Chem. Eur. J.,* 2009, vol. 15, 4857-4864
- *Phys.Chem.Chem. Phys.,* 2012, vol. 14, 2921-2928
- **C. W. TANG.** *Appl. Phys. Lett.,* 1986, vol. 48 (2), 183-185
- **N. KARL ; A. BAUER ; J. HOLZÄPFEL ; J. MARKTANNER ; M. MÖBUS ; F. STÖLZLE.** *Mol. Cryst. Liq. Cryst.,* 1994, vol. 252, 243-258
- **C. J. BRABEC ; N. S. SARICIFTCI ; J. C. HUMMELEN.** *Adv. Funct. Mater.,* 2001, vol. 11 (1), 15
- **J. XUE ; B. P. RAND ; S. UCHIDA ; S. R. FORREST.** *J. Appl. Phys.,* 2005, vol. 98, 124903
- **B. J. DRECHSEL et al.** *Org. Electron.,* 2004, vol. 5 (4), 175
- **MAENNIG et al.** *Appl. Phys. A,* 2004, vol. 79, 1-14
- **P. PEUMANS ; A. YAKIMOV ; S. R. FORREST.** *J. Appl. Phys,* 2003, vol. 93 (7), 3693-3723
- **J. DRECHSEL et al.** *Thin Solid Films,* 2004, vol. 451452, 515-517
- **B. FACCIETTI.** *Adv. Mat.,* 2005, vol. 17, 1705-1725
- *Angew. Chem.,* 2007, vol. 119, 3824-3827